# EUROPEAN PATENT SPECIFICATION

(11) **EP 1 147 101 B1**
(45) Date of publication and mention of the grant of the patent: **20.11.2002**
(21) Application number: 00905758.9
(22) Date of filing: 27.01.2000
(51) Int. Cl.: C07D 317/28, C07D 307/14, C07D 207/48, C07D 207/09, C07D 263/04, A61K 31/335, A61P 35/04

(54) **REVERSE HYDROXAMATE INHIBITORS OF MATRIX METALLOPROTEINASES**
UMGEKEHRT HYDROXAMAT INHIBITOREN VON MATRIX METALLOPROTEINASEN
COMPOSES D'HYDROXAMATE INVERSE UTILISES COMME INHIBITEURS DE METALLOPROTEASES MATRICIELLES

(30) Priority: 27.01.1999 US 239087
(43) Date of publication of application: 24.10.2001
(73) Proprietor: ABBOTT LABORATORIES, Abbott Park, IL 60064-6050 (US)
(72) Inventor: CURTIN, Michael, L., Pleasant Prairie, WI 53158 (US); DAI, Yujia, Gurnee, IL 60031 (US); DAVIDSEN, Steven, K., Libertyville, IL 60048 (US); HEYMAN, Howard, R., Chicago, IL 60641 (US); HOLMES, James, H., Gurnee, IL 60031 (US); MICHAELIDES, Michael, R., Libertyville, IL 60048 (US); STEINMAN, Douglas, H., Morton Grove, IL 60053 (US)
(74) Representative: Modiano, Guido, Dr.-Ing.
(86) International application number: US0002038
(87) International publication number: WO00044739

(56) References cited:
- EP-A- 0 780 386
- S.WNUK ET AL.: "NUCLEIC ACID RELATED COMPOUNDS.67." CANADIAN JOURNAL OF CHEMISTRY., vol. 69, no. 12, 1991, pages 2104-11, XP002140818 NATIONAL RESEARCH COUNCIL. OTTAWA., CA ISSN: 0008-4042

## Description

### Technical Field

This invention relates to compounds having activity to inhibit matrix metalloproteinases, to pharmaceutical compositions comprising these compounds, and to a medical method of treatment. More particularly, this invention concerns reverse hydroxamate-containing compounds which inhibit matrix metalloproteinases, pharmaceutical compositions comprising the compounds, and methods of inhibiting matrix metalloproteinases in a mammal.

### Background of the Invention

EP 0 780 386 A discloses compounds containing the -S(O)ₙ- group, which are inhibitors of metalloproteases.

The matrix metalloproteinases (MMP's) are a class of extracellular enzymes including collagenase, stromelysin, and gelatinase which are believed to be involved in the tissue destruction which accompanies a large number of disease states varying from arthritis to cancer.

Typical connective tissue cells are embedded within an extracellular matrix of high molecular weight proteins and glycoproteins. In healthy tissue, there is a continual and delicately-balanced series of processes which include cell division, matrix synthesis and matrix degradation. In certain pathological conditions, an imbalance of these three processes can lead to improper tissue restructuring. In arthritis, for example, joint mobility can be lost when there is improper remodeling of load-bearing joint cartilage. With cancer, lack of coordination of cell division and the two processes of matrix synthesis and degradation may lead to conversion of transformed cells to invasive phenotypes in which increased matrix turnover permits tumor cells to penetrate basement membranes surrounding capillaries which, in turn, may lead to subsequent metastasis.

There has been heightened interest in discovering therapeutic agents which bind to and inhibit MMP's. The discovery of new therapeutic agents possessing this activity will lead to new drugs having a novel mechanism of action for combating disease states involving tissue degenerative processes including, for example, rheumatoid arthritis, osteoarthritis, osteopenias such as osteoporosis, periodontitis, gingivitis, corneal, epidermal or gastric ulceration, and tumor growth and metastasis or invasion.

This invention discloses a series of MMP inhibitors having a unique combination of potency, pharmacokinetics, and fewer side effects.

### Summary of the Invention

In its principle embodiment, the present invention provides a matrix metalloproteinase inhibitory compound of formula (I), 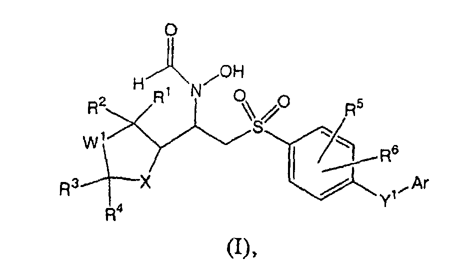 or a pharmaceutically acceptable salt or prodrug thereof, wherein
**W**^{**1**} is selected from the group consisting of
(1) -O-,
(2) -CH₂O-,
   and
(3) -CH₂-;
wherein each group is drawn with its left-hand end being the end which attaches to the carbon containing R¹ and R², and its right-hand end being the end which attaches to the carbon containing R³ and R⁴;
**X** is selected from the group consisting of
(1) -O-,
   and
(2) -N(R⁷)-, wherein R⁷ is selected from the group consisting of
   (a) hydrogen,
   (b) alkyl,
   (c) -SO₂-alkyl,
      and
   (d) alkanoyl;
**R**^{**1**} and **R**^{**2**} are independently selected from the group consisting of
(1) hydrogen,
(2) alkyl,
   and
(3) hydroxyalkyl;
**R**^{**3**} and **R**^{**4**} are independently selected from the group consisting of
(1) hydrogen,
   and
(2) alkyl,
or
**R**^{**3**} and **R**^{**4**} taken together are oxo,
or
**R**^{**3**} and **R**^{**4**}**,** taken together with the carbon atom to which they are attached, form a cycloalkyl ring;
**R**^{**5**} and **R**^{**6**} are independently selected from the group consisting of
(1) hydrogen,
(2) alkyl,
(3) perfluoroalkyl,
(4) halo,
(5) haloalkyl,
(6) alkoxy,
(7) hydroxy,
(8) hydroxyalkyl,
(9) alkoxyalkyl,
   and
(10) nitro;
**Y**^{**1**} is selected from the group consisting of
(1) a covalent bond,
(2) -O-,
(3) alkylene of two to four carbon atoms,
(4) piperidineneyl,
(5) alkenylene of two carbon atoms,
(6) alkynylene of two carbon atoms,
(7) -SO₂-,
   and
(8) -C(O)-;
**Ar** is selected from the group consisting of
(1) phenyl,
(2) pyridyl,
(3) pyrazinyl,
(4) pyridazinyl,
(5) furyl,
(6) thienyl,
(7) isoxazolyl,
(8) oxazolyl,
(9) thiazolyl,
   and
(10) isothiazolyl,
wherein (1)-(10) can be optionally substituted with one, two, or three substituents independently selected from the group consisting of
(a) alkyl,
(b) alkoxy, wherein the alkoxy can be optionally substituted with alkoxy,
(c) -(alkylene)-CO₂R⁸, wherein R⁸ is either hydrogen or alkyl,
(d) -(alkylene)-NR⁹R¹⁰, wherein R⁹ and R¹⁰ are independently selected from the group consisting of
   (i) alkyl,
   (ii) phenyl,
      and
   (iii) phenylalkyl,
      wherein for (ii) and (iii), the phenyl and the phenyl part of the phenylalkyl can be optionally substituted with one or two substituents independently selected from the group consisting of halo and alkoxy,
(e) alkoxyalkyl,
(f) cyano,
(g) cyanoalkyl,
(h) halo,
(i) haloalkyl,
(j) hydroxy,
(k) hydroxyalkyl,
(l) thioalkoxy,
(m) thioalkoxyalkyl,
(n) phenylalkoxy,
(o) phenoxy,
(p) phenoxyalkyl,
(q) (heterocycle)oxy,
(r) (heterocycle)oxyalkyl,
(s) perfluoroalkyl,
(t) perfluoroalkoxy,
(u) sulfinylalkyi,
(v) sulfonylalkyl,
(w) 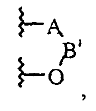 wherein A is selected from the group consisting of -CH₂-, -CH₂O- and -O-, and B¹ is selected from the group consisting of -C(O)- and -(C(R")₂)ᵥ -, wherein R" is either hydrogen or alkyl, and v is 1-3,
   and
(x) -N(R⁸)SO₂R¹¹, wherein R¹¹ is selected from the group consisting of
   (i) hydrogen,
   (ii) alkyl,
      and
   (iii) -N(R⁸)(R¹²), wherein R¹² is hydrogen or alkyl,
   wherein for (q) and (r), the heterocycle part of the (heterocycle)oxy and the (heterocycle)oxyalkyl are selected from the group consisting of
   (i) pyridyl,
   (ii) pyrazinyl,
   (iii) pyridazinyl,
   (iv) furyl,
   (v) thienyl,
   (vi) isoxazolyl,
   (vii) oxazolyl,
   (viii) thiazoloyl,
      and
   (ix) isothiazolyl,
   and wherein for (q) and (r), the heterocycle part of the (heterocycle)oxy and the (heterocycle)oxyalkyl can be optionally substituted with one or two substituents independently selected from the group consisting of
   (i) alkyl,
   (ii) alkoxy,
   (iii) perfluoroalkyl,
   (iv) halo,
   (v) cyano,
   (vi) cyanoalkyl,
   (vii) haloalkyl,
      and
   (viii) alkanoyl,
   and
   wherein for (o) and (p), the phenyl part of the phenoxy and the phenoxyalkyl can be optionally substituted with one or two substituents independently selected from the group consisting of
   (i) alkyl,
   (ii) alkoxy,
   (iii) perfluoroalkyl,
   (iv) halo,
   (v) cyano,
   (vi) cyanoalkyl,
   (vii) haloalkyl,
      and
   (viii) alkanoyl.

Preferred compounds of the invention are those wherein both X and W¹ of formula I are oxygen.

In another embodiment, the present invention provides pharmaceutical compositions which comprise a therapeutically effective amount of compound of formula I in combination with a pharmaceutically acceptable carrier.

In yet another embodiment, the present invention provides a method of inhibiting matrix metalloproteinases in a mammal in recognized need of such treatment comprising administering to the mammal a therapeutically effective amount of a compound of formula I.

### Detailed Description of the Invention

As used throughout this specification and the appended claims, the following terms have the meanings specified:

The term "alkanoyl," as used herein, represents an alkyl group attached to the parent molecular moiety through a carbonyl group.

The term "alkenylene," as used herein, represents a divalent group derived from a straight or branched chain hydrocarbon containing at least one double bond.

The term "alkoxy," as used herein, represents an alkyl group attached to the parent molecular moiety through an oxygen atom. The alkoxy groups of this invention can be optionally substituted.

The term "alkoxyalkyl," as used herein, represents an alkoxy group attached to the parent molecular moiety through an alkylene group.

The term "alkyl," as used herein, represents a saturated straight or branched chain hydrocarbon radical.

The term "alkylene," as used herein, represents a saturated divalent hydrocarbon group derived from a straight or branched chain saturated hydrocarbon by the removal of two hydrogen atoms.

The term "alkynylene," as used herein, represents a divalent group derived from a straight or branched chain hydrocarbon containing at least one triple bond.

The term "cyano," as used herein, represents -CN.

The term "cyanoalkyl," as used herein, represents acyano group attached to the parent molecular moiety through an alkyl group.

The term "halo," as used herein, represents -F, -Cl, -Br, and -I.

The term "haloalkyl," as used herein, represents an alkyl group substituted by one, two, three, or four halogen atoms.

The term "heterocycle," as used herein, represents a five-, six-, or seven-membered ring containing one, two, or three heteroatoms independently selected from the group consisting of nitrogen, oxygen, and sulfur. The five-membered ring has zero to two double bonds and the six- and seven-membered rings have zero to three double bonds. The heterocycle groups of this invention can be optionally substituted.

The term "(heterocycle)oxy," as used herein, represents a heterocycle group attached to the parent molecular moiety through an oxygen atom, The (heterocycle)oxy groups of this invention can be optionally substituted.

The term "(heterocycle)oxyalkyl," as used herein, represents a (heterocycle)oxy group attached to the parent molecular moiety through an alkyl group. The (heterocycle)oxyalkyl groups of this invention can be optionally substituted.

The term "hydroxy," as used herein, represents -OH.

The term "hydroxyalkyl," as used herein, represents ahydroxy group attached to the parent molecular moiety through an alkyl group.

The term "nitro," as used herein, represents -NO₂.

The term "oxo," as used herein, represents (=O).

The term "perfluoroalkoxy," as used herein, represents a perfluoroalkyl group attached to the parent molecular moiety through an oxygen atom.

The term "perfluoroalkyl," as used herein, represents an alkyl group wherein each hydrogen radical bound to the alkyl group has been replaced by a fluoride radical.

The term "pharmaceutically acceptable prodrug," as used herein, represents those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of this invention.

The term "pharmaceutically acceptable salt," as used herein, represents salts which are, within the scope of sound medical judgment, suitable for use in contact with the tissues of humans and lower animals without undue toxicity, irritation, allergic response and the like and are commensurate with a reasonable benefit/risk ratio. The salts can be prepared *in situ* during the final isolation and purification of the compounds of the invention or separately by reacting the free base group with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, trifluoroacetate, undecanoate, valerate salts and the like. Representative alkali or alkaline earth metal salts include sodium, lithium, potassium, calcium, magnesium and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine and the like.

The term "phenoxy," as used herein, represents a phenyl group attached to the parent molecular moiety through an oxygen atom. The phenoxy groups of this invention can be optionally substituted.

The term "phenoxyalkyl," as used herein, represents a phenoxy goup attached to the parent molecular moiety through an alkyl group. The phenoxyalkyl groups of this invention are optionally substituted.

The term "phenylalkoxy," as used herein, represents a phenyl group attached to the parent molecular moiety through an alkoxy group.

The term "phenylalkyl," as used herein, represents a phenyl group attached to the parent molecular moiety through an alkyl group. The phenylalkyl groups of this invention can be optionally substituted.

The term "prodrug," as used herein, represents compounds which are rapidly transformed *in vivo* to parent compounds defined above, such as, by hydrolysis in blood.

The term "sulfinyl," as used herein, represents -S(O)-.

The term "sulfinylalkyl," as used herein, represents an alkyl group attached to the parent molecular moiety through a sulfinyl group.

The term "sulfonyl," as used herein, represents -SO₂-.

The term "sulfonylalkyl," as used herein, represents an alkyl group attached to the parent molecular moiety through a sulfonyl group.

The term "thioalkoxy," as used herein, represents an alkyl group attached to the parent molecular moiety through a sulfur atom.

The term "thioalkoxyalkyl," as used herein, represents a thioalkoxy group attached to the parent molecular moiety through an alkyl group.

Compounds of the present invention can exist as stereoisomers, wherein asymmetric or chiral centers are present. These compounds are designated by the symbols "R" or "S," depending on the configuration of substituents around the chiral carbon atom. The present invention contemplates various stereoisomers and mixtues thereof. Stereoisomers include enantiomers and diastereomers, and mixtures of enantiomers or diastereomers are designated (RS). Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

In accordance with methods of treatment and pharmaceutical compositions of the invention, the compounds can be administered alone or in combination with other matrix metalloproteinase inhibiting agents. When using the compounds, the specific therapeutically effective dose level for any particular patient will depend upon factors such as the disorder being treated and the severity of the disorder; the activity of the particular compound used; the specific composition employed; the age, body weight, general health, sex, and diet of the patient; the time of administration; the route of administration; the rate of excretion of the compound employed; the duration of treatment; and drugs used in combination with or coincidently with the compound used. The compounds can be administered orally, parenterally, osmotically (nasal sprays), rectally, vaginally, or topically in unit dosage formulations containing carriers, adjuvants, diluents, vehicles, or combinations thereof. The term "parenteral" includes infusion as well as subcutaneous, intravenous, intramuscular, and intrastemal injection.

Parenterally adminstered aqueous or oleaginous suspensions of the compounds can be formulated with dispersing, wetting, or suspending agents. The injectable preparation can also be an injectable solution or suspension in a diluent or solvent. Among the acceptable diluents or solvents employed are water, saline, Ringer's solution, buffers, monoglycerides, diglycerides, fatty acids such as oleic acid, and fixed oils such as monoglycerides or diglycerides.

The inhibitory effect of parenterally administered compounds can be prolonged by slowing their absorption. One way to slow the absorption of a particular compound is adminstering injectable depot forms comprising suspensions of crystalline, amorphous, or otherwise water-insoluble forms of the compound. The rate of absorption of the compound is dependent on its rate of dissolution which is, in turn, dependent on its physical state. Another way to slow absorption of a particular compound is administering injectable depot forms comprising the compound as an oleaginous solution or suspension. Yet another way to slow absorption of a particular compound is administering injectable depot forms comprising microcapsule matrices of the compound trapped within liposomes, microemulsions, or biodegradable polymers such as polylactide-polyglycolide, polyorthoesters or polyanhydrides. Depending on the ratio of drug to polymer and the composition of the polymer, the rate of drug release can be controlled.

Transdermal patches can also provide controlled delivery of the compounds. The rate of absorption can be slowed by using rate controlling membranes or by trapping the compound within a polymer matrix or gel. Conversely, absorption enhancers can be used to increase absorption.

Solid dosage forms for oral administration include capsules, tablets, pills, powders, and granules. In these solid dosage forms, the active compound can optionally comprise diluents such as sucrose, lactose, starch, talc, silicic acid, aluminum hydroxide, calcium silicates, polyamide powder, tableting lubricants, and tableting aids such as magnesium stearate or microcrystalline cellulose. Capsules, tablets and pills can also comprise buffering agents, and tablets and pills can be prepared with enteric coatings or other release-controlling coatings. Powders and sprays can also contain excipients such as talc, silicic acid, aluminum hydroxide, calcium silicate, polyamide powder, or mixtures thereof. Sprays can additionally contain customary propellants such as chlorofluorohydrocarbons or substitutes therefor.

Liquid dosage forms for oral administration include emulsions, microemulsions, solutions, suspensions, syrups, and elixirs comprising inert diluents such as water. These compositions can also comprise adjuvants such as wetting, emulsifying, suspending, sweetening, flavoring, and perfuming agents.

Topical dosage forms include ointments, pastes, creams, lotions, gels, powders, solutions, sprays, inhalants, and transdermal patches. The compound is mixed under sterile conditions with a carrier and any needed preservatives or buffers. These dosage forms can also include excipients such as animal and vegetable fats, oils, waxes, paraffins, starch, tragacanth, cellulose derivatives, polyethylene glycols, silicones, bentonites, silicic acid, talc and zinc oxide, or mixtures thereof. Suppositories for rectal or vaginal administration can be prepared by mixing the compounds with a suitable nonirritating excipient such as cocoa butter or polyethylene glycol, each of which is solid at ordinary temperature but fluid in the rectum or vagina. Ophthalmic formulations comprising eye drops, eye ointments, powders, and solutions are also contemplated as being within the scope of this invention.

The total daily dose of the compounds administered to a host in single or divided doses can be in amounts from about 0.1 to about 200 mg/kg body weight or preferably from about 0.25 to about 100 mg/kg body weight. Single dose compositions can contain these amounts or submultiples thereof to make up the daily dose.

Preferred compounds of the invention are those wherein both X and W¹ of formula I are oxygen.

Additional preferred embodiments are:
compounds of formula (I), wherein R⁵ and R⁶ are hydrogen,
compounds of formula (I), wherein Y¹ is -O-,
   and
compounds of formula (I), wherein Y¹ is a covalent bond.

Most preferred is the compound of Example 1.

Specific compounds of the instant invention include, but are not limited to, (1S)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4R)-2,2-dimethyl-1,3-dioxolan-4-yl)-2-((4-(4-(trifluoromethoxy)phenoxy)phenyl)sulfonyl)ethyl(hydroxy)formamide, (1S)-1-((4R)-2,2-dimethyl-1,3-dioxolan-4-yl)-2-((4-(4-(trifluoromethoxy)phenoxy)phenyl)sulfonyl)ethyl(hydroxy)formamide, 4-chloro-4'-(((2S)-2-((4S)-2,2-dimethyl-1,3-dioxolan-4-yl)-2-(formyl(hydroxy)amino)ethyl)sulfonyl)-1,1'-biphenyl, (1S)-1-((4S)-2,2-diethyl-1,3-dioxolan-4-yl)-2-((4-(4-(trifluoromethoxy)phenoxy)phenyl)sulfonyl)ethyl(hydroxy)formamide, 1,4,5-trideoxy-4-(formyl(hydroxy)amino)-2,3-O-(1-methylethylidene)-5-((4-(4-(trifluoromethoxy)phenoxy)phenyl)sulfonyl)-D-xylitol, (1S)-1-((4S)-2,2-dimethyl-1,3-dioxolan-4-yl)-2-((4-(4-methoxyphenoxy)phenyl)sulfonyl)ethyl(hydroxy)formamide, (1S)-2-((4-(4-chlorophenoxy)phenyl)sulfonyl)-1-((4S)-2,2-dimethyl-1,3-dioxolan-4-yl)ethyl(hydroxy)formamide, (1S)-1-((4S)-2,2-dimethyl-1,3-dioxolan-4-yl)-2-((4-(4-(trifluoromethyl)phenoxy)phenyl)sulfonyl)ethyl(hydroxy)formamide, (1S)-1-((4S)-2,2-dimethyl-1,3-dioxolan-4-yl)-2-((4-(4-methylphenoxy)phenyl)sulfonyl)ethyl(hydroxy)formamide, 4-(((2S)-2-((4S)-2,2-dimethyl-1,3-dioxolan-4-yl)-2-(formyl(hydroxy)amino)ethyl)sulfonyl)-4'-(trifluoromethyl)-1,1'-biphenyl, (1S)-1-((4S)-1,3-dioxolan-4-yl)-2-((4-(4-(trifluoromethoxy)phenoxy)phenyl)sulfonyl)ethyl(hydroxy)formamide, 4-(((2S)-2-((4S)-2,2-dimethyl-1,3-dioxolan-4-yl)-2-(formyl(hydroxy)amino)ethyl)sulfonyl)-4'-(2-methoxyethoxy)-1,1'-biphenyl, (1S)-1-((4S)-2,2-dimethyl-1,3-dioxolan-4-yl)-2-((4-(4-(2-methoxyethoxy)phenoxy)phenyl)sulfonyl)ethyl(hydroxy)formamide, 1,2,4-trideoxy-2-(formyl(hydroxy)amino)-4,4-dimethyl-3,5-O-(1-methylethylidene)-1-((4-(4-(trifluoromethoxy)phenoxy)phenyl)sulfonyl)-D-threo-pentitol, hydroxy((1S)-1-((2R)-tetrahydro-2-furanyl)-2-((4-(4-(trifluoromethoxy)phenoxy)phenyl)sulfonyl)ethyl)formamide, hydroxy((1R)-1-((2R)-tetrahydro-2-furanyl)-2-((4-(4-(trifluoromethoxy)phenoxy)phenyl)sulfonyl)ethyl)formamide, hydroxy(( 1S)-1-((2R)-1-(methylsulfonyl)pyrrolidinyl)-2-((4-(4-(trifluoromethoxy)phenoxy)phenyl)sulfonyl)ethyl)formamide, hydroxy(( 1RS)-1-((4S)-2-oxo-1,3-oxazolidin-4-yl)-2-((4-(4-(trifluoromethoxy)phenoxy)phenyl)sulfonyl)ethyl)formamide, hydroxy((1RS)-1-((4S)-3-methyl-2-oxo-1,3-oxazolidin-4-yl)-2-((4-(4-(trifluoromethoxy)phenoxy)phenyl)sulfonyl)ethyl)formamide, and 1,2-dideoxy-2-(formyl(hydroxy)amino)-3,4-O-(1-methylethylidene)-1-((4-(4-(trifluoromethoxy)phenoxy)phenyl)sulfonyl)-L-threo-pentitol.

A more preferred compound for the practice of the instant invention is (1S)-1-((4S)-2,2-dimethyl-1,3-dioxolan-4-yl)-2-((4-(4-(trifluoromethoxy)phenoxy)phenyl)sulfonyl)ethyl(hydroxy)formamide.

### Determination of Biological Activity

The efficacy of the compounds of the invention as matrix metalloproteinase inhibitors was determined by measuring inhibition as outlined below for Gelatinase A, a member of this family of enzymes. Recombinant active gelatinase-A (MMP-2) is purchased from Oncogene Research. The enzyme is assayed by its cleavage of a fluorescent substrate in 150 µL volume in a microfluor plate as described in *Science* **1990**, *247*, 954-958. Upon cleavage of the substrate, the fluorescence of the EDANS group is increased 30-fold, and this increase is monitored using a f-max (Molecular Devices) fluorescent plate reader (ex: 335 nm; em: 485 nm). The rates of cleavage of the substrate by gelatinase-A in the presence or absence of inhibitors are measured in a 40 min assay at ambient temperature. Stock solutions of the compounds in DMSO are prepared, and these solutions are diluted into the assay buffer (50 mM Tris HCl, pH 7.4, with 150 mM NaCl and 10 mM CaCl₂), which is also used for dilution of the enzyme and substrate. The potencies of the compounds [IC₅₀], shown below in Table 1, are calculated by plotting the logit function of the percent inhibition data relative to control vs. the logarithm of the inhibitor concentrations.

**Table 1:**

| MMP-2 Inhibition | |
|---|---|
| Example | IC₅₀ (nM) |
| 1 | 0.8 |
| 8 | 0.3 |
| 9 | 0.5 |
| 11 | 0.8 |
| 13 | 0.2 |
| 14 | 0.4 |
| 15 | 0.3 |
| 17 | 0.6 |
| 20 | 1.4 |
| 21 | 0.9 |

### Synthetic Methods

Abbreviations which have been used in the descriptions of the schemes and the examples that follow are: DMSO for dimethylsulfoxide; MTBE for methyl tert-butyl ether, THF for tetrahydrofuran, and DMF for N,N-dimethylformamide.

The compounds and processes of the present invention will be better understood in connection with the following synthetic scheme which illustrates methods by which the compounds of the invention can be prepared. The compounds can be prepared by a variety of synthetic routes. Representative procedures are shown in Scheme 1. The groups R¹, R², R³, R⁴, R⁵, and R⁶ are defined above. It will be readily apparent to one of ordinary skill in the art that the compounds defined above can be synthesized by substitution of the appropriate reactants and agents in the syntheses shown below.

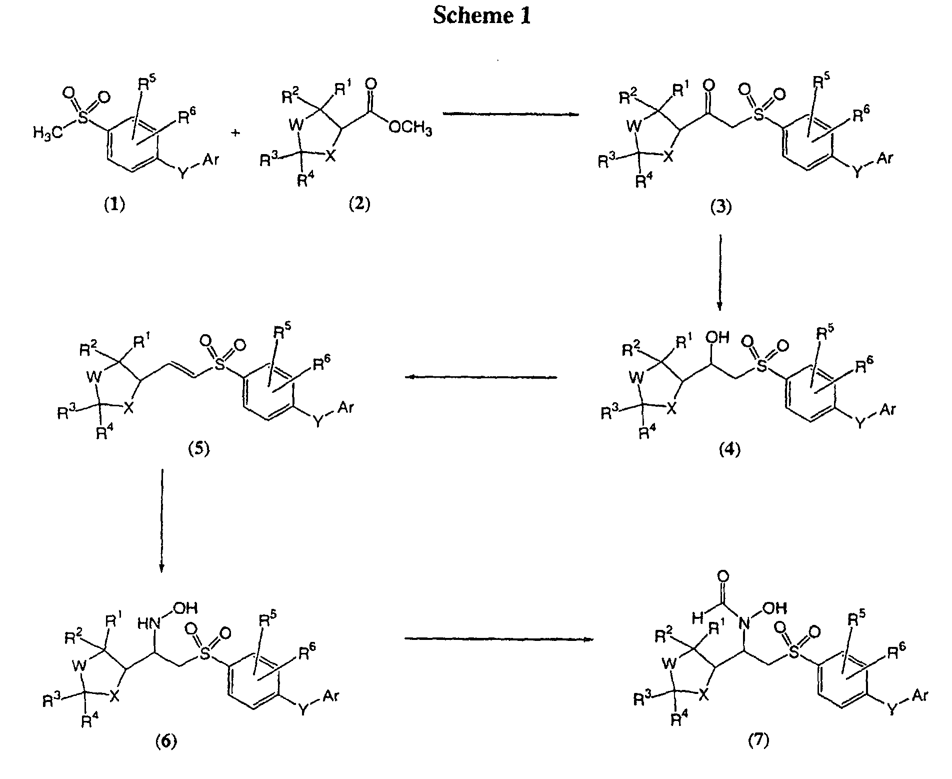

As shown in Scheme 1, compounds of formula (**1**) can be converted to compounds of formula (**3**) by treatment with base followed by condensation with compounds of formula (**2**). Representative bases include n-butyllithium, tert-butyllithium, lithium hexamethyldisilazide, and lithium diisopropylamide. Examples of solvents used in these reactions include THF, diethyl ether, toluene, hexanes, or mixtures thereof. The reaction temperature is about -100 °C to 30 °C and depends on the method chosen. Reaction times are typically 0.5 to 8 hours. In a preferred embodiment, compounds of formula (**1**) are treated with n-butyllithium in THF at -78 °C, then treated with compounds of formula (**2**) in THF at -78°C for 3 hours to provide compounds of formula (**3**).

Compounds of formula (3) can be converted to compounds of formula (**4**) by treatment with a reducing agent. Representative reducing agents include sodium borohydride, diisobutylaluminum hydride, lithium tri-tert-butoxyaluminohydride, and sodium triacetoxyborohydride. Examples of solvents used in these reactions include toluene, hexanes, THF, ethanol, methanol, or mixtures thereof. The reaction temperature is about -78 °C to 60 °C, and depends on the method chosen. Reaction times are typically about 15 minutes to 12 hours. In a preferred embodiment, compounds of formula (**3**) are treated with sodium borohydride in ethanol at room temperature for 0.5 hours to provide compounds of formula (**4**).

Conversion of compounds of formula (**4**) to compounds of formula (**5**) can be accomplished by treatment with an acylating agent in the presence of excess base. Representative acylating agents include methanesulfonyl chloride, p-toluenesulfonyl chloride, trifluoracetic anhydride, and benzenesulfonyl chloride. Examples of bases include triethylamine, diisopropylethylamine, and pyridine. Representative solvents used in these reactions include dichloromethane, carbon tetrachloride, chloroform, THF, and diethyl ether. The reaction temperature is about -40 °C to 100 °C, and depends on the method chosen. Reaction times are typically 0.5 to 24 hours. In a preferred embodiment, compounds of formula (**4**) are treated with methanesulfonyl chloride and excess triethylamine in dichloromethane at 0 °C for 15 minutes, then warmed to room temperature for 1 hour to provide compounds of formula (**5**).

Compounds of formula (**5**) can be converted to compounds of formula (**6**) by treatment with hydroxylamine. Examples of solvents used in this reaction include THF, diethyl ether, water, dioxane, or mixtures thereof. The reaction temperature is about -78 °C to 30 °C and depends on the method chosen. Reaction times are typically 0.5 to 24 hours. In a preferred embodiment, compounds of formula (**5**) are treated with aqueous hydroxylamine in THF at -35°C and warmed to 0°C over 4 hours to provide compounds of formula (**6**).

Conversion of compounds of formula (**6**) to compounds of formula (**7**) can be accomplished by treatment with 2,2,2-trifluoroethyl formate or formic acetic anhydride. Examples of solvents used in these reactions includes MTBE, diethyl ether, THF, dichloromethane, and dioxane. The reaction temperature is about 25°C to 120°C and depends on the method chosen. Reaction times are typically 2 to 36 hours. In a preferred embodiment, compounds of formula (**6**) are treated with 2,2,2-trifluorethyl formate in MTBE at reflux for 20 hours to provide compounds of formula (**7**).

The instant invention will now be described in connection with certain preferred embodiments which are not intended to limit its scope. On the contrary, the instant invention covers all alternatives, modifications, and equivalents as can be included within the scope of the claims. Thus, the following examples, which include preferred embodiments, will illustrate the preferred practice of the instant invention, it being understood that the examples are for the purposes of illustration of certain preferred embodiments and are presented to provide what is believed to be the most useful and readily understood description of its procedures and conceptual aspects.

### Example 1

### (1S)-1-((4S)-2,2-dimethyl-1,3-dioxolan-4-yl)-2-((4-(4-(trifluoromethoxy)phenoxy)phenyl)sulfonyl)ethyl(hydroxy)formamide

### Example 1A

### 4-(4'-trifluoromethoxyphenoxy)phenyl methylsulfone

A mixture of anhydrous potassium carbonate (159.0 g, 1.15 mol), 4-trifluoromethoxyphenol (150 mL, 1.16 mol), and 4-fluorophenyl methyl sulfone (200.0 g, 1.15 mol) in DMSO (1.5 L) was heated to 120 °C and stirred vigorously for 18 hours. The mixture was cooled to room temperature, filtered through a glass wool plug with MTBE, and concentrated. The concentrate was diluted with water (1 L), and cooled to 0°C. The resulting precipitate was collected by filtration, washed with water, and dried under vacuum at 50 °C. Recrystallization from MTBE/hexanes provided the desired product.
mp: 71.5-72 °C.

### Example 1B

### 1-((4R)-2,2-dimethyl-1,3-dioxolan-4-yl)-2-((4-(4-(trifluoromethoxy)phenoxy)phenyl)sulfonyl)ethanone

A solution of 4-(4'-trifluoromethoxyphenoxy)phenyl methyl sulfone (36.6 g, 0.11 mol) in THF (600 mL) at -78 °C was treated with n-butyllithium (2.5M in hexanes, 48.0 mL, 0.12 mol) over 5 minutes and stirred for 1 hour. The solution was transferred by cannula to a -78 °C solution of methyl (4R)-2,2-dimethyl-1,3-dioxolane-4-carboxylate (19.6 g, 0.12 mol) in THF (400 mL) over 30 minutes, stirred for 3 hours, treated with 1M H₂SO₄ (75 mL), and warmed to 0 °C. The aqueous phase was extracted with MTBE (500 mL), and the combined organic phases were washed with water and brine, dried (Na₂SO₄), and filtered. The solution was passed through a pad of silica gel (100 g), the pad was washed with MTBE, and the resulting solution was concentrated to 1/5 the original volume, treated with hexanes (300 mL), and cooled to room temperature. The resulting precipitate was collected by filtration, washed with MTBE/hexanes, and dried to provide the desired product.
mp: 80-81 °C; (α)_{D} + 49.9 ° (c 4.1, CH₂Cl₂).

### Example 1C

### (4S)-2,2-dimethyl-4-((EZ)-2-((4-(4-(trifluoromethoxy)phenoxy)phenyl)sulfonyl)ethenyl)-1,3-dioxolane

A suspension of Example 1B (37.3 g, 81 mmol) in ethanol (250 mL) at room temperature was treated with sodium borohydride (1.40 g, 37 mmol), stirred for 30 minutes, treated dropwise with acetic acid (1 mL), and concentrated. The concentrate was partitioned between ethyl acetate and water, and the organic phase was washed sequentially with 1 M NaHCO₃, water, and brine, dried (Na₂SO₄), and filtered. The solution was passed through a pad of silica gel (200 g) using 3:2/hexanes:ethyl acetate, concentrated, redissolved in CH₂Cl₂ (250 mL), treated with triethylamine (31.1 mL, 222 mmol), cooled to 0 °C, and treated with methanesulfonyl chloride (8.0 mL, 103 mmol) over 40 minutes. The mixture was stirred for 15 minutes, warmed to room temperature, stirred for 1 hour, washed sequentially with water, 1M HCl, water, 1M NaHCO₃, water, and brine, dried (Na₂SO₄), and filtered. The concentrate was purified by flash column chromatography on silica gel using 98:2/dichloromethane:ethyl acetate. The purified concentrate was recrystallized from MTBE/hexanes to provide the desired product as a mixture of cis- and trans-isomers.
mp: 67-71 °C.

### Examples 1D and 1E

### (4S)-4-((1S)-1-(hydroxyamino)-2-((4-(4-(trifluoromethoxy)phenoxy)phenyl)sulfonyl)ethyl)-2,2-dimethyl-1,3-dioxolane and

### (4S)-4-((1R)-1-(hydroxyamino)-2-((4-(4-(trifluoromethoxy)phenoxy)phenyl)sulfonyl)ethyl)-2,2-dimethyl-1,3-dioxolane

A solution of Example 1C (28.6 g, 64.3 mmol) in THF (800 mL) at -35 °C was treated with 50% aqueous hydroxylamine (6.4 g, 193 mmol), warmed to 0 °C over 4 hours, and concentrated. The concentrate was dissolved in MTBE, washed with water and brine, dried (Na₂SO₄), filtered, and concentrated. The concentrate was recrystallized from MTBE/hexanes to provide a mixture of two diastereomers, which were separated by flash column chromatography on silica gel using 70:30/hexanes:ethyl acetate to provide the desired products.

### Example 1F

### (1S)-1-((4S)-2,2-dimethyl-1,3-dioxolan-4-yl)-2-((4-(4-(trifluoromethoxy)phenoxy)phenyl)sulfonyl)ethyl(hydroxy)formamide

A mixture of 2,2,2-trifluoroethanol (500 mL) and formic acid (95-97%, 1140 mL) at 75 °C was stirred for 16 hours, then distilled (64-66 °C) to provide 2,2,2-trifluoroethyl formate (TFE-F reagent) as an 8.9M solution.
¹H NMR (300 MHz, CDCl₃): δ 4.55 (q, 2H), 8.12 (s, 1H).

A solution of Example 1D (21.6 g, 45.2 mmol) in MTBE (200 mL) was treated with TFE-F reagent (8.9M, 50 mL, 443 mmol), heated to reflux, stirred for 20 hours, and slowly cooled to room temperature. The mixture was cooled to 0 °C, and the resulting precipitate was collected by filtration, washed with cold MTBE, and dried to provide the desired product.
mp: 127.5-128.5 °C; (α)_{D}+ 4.85 ° (c 2.14, CH₂Cl₂);
MS (APCI) *m*/*z* 506 (M+H)⁺;
¹H NMR (300 MHz, DMSO-d₆): δ 1.20 (s, 1.2H), 1.23 (s, 1.8H), 1.26 (s, 1.2H), 1.30 (s, 1.8H), 3.32 (t, 0.6H, J=7.5 Hz), 3.59-3.76 (m, 2.1H), 3.92-4.15 (m, 3H), 4.57 (t, 0.4H, J=8.4 Hz), 7.18-7.32 (m, 4H), 7.48 (d, 2H, J=9.6 Hz), 7.82 (s, 0.6H), 7.88 (d, 0.8H, J=9.6 Hz), 7.94 (d, 1.2H, J=9.6 Hz), 8.13 (s, 0.4H), 9.63 (s, 0.6H), 10.00 (s, 0.4 H);
Anal. Calcd. for C₂₁H₂₂NO₈SF₃: C, 49.90; H, 4.39; N, 2.77; S, 6.34. Found: C, 49.88; H, 4.24; N, 2.76; S, 6.43.

### Example 2

### (1R)-1-((4S)-2,2-dimethyl-1,3-dioxolan-4-yl)-2-((4-(4-(trifluoromethoxy)phenoxy)phenyl)sulfonyl)ethyl(hydroxy)formamide

The desired product was prepared by substituting Example 1E for Example 1D in Example 1F.
mp: 149-150°C;
MS (ESI) *m*/*z* 506 (M+H)⁺;
¹H NMR (300 MHz, DMSO-d₆): δ 1.04 (s, 1.5H), 1.13 (s, 1.5H), 1.20 (s, 1.5H), 1.23 (s, 1.5H), 3.57-4.11 (m, 5.5H), 4.39 (t, 0.5H, J=9.80 Hz), 7.19-7.30 (m, 4H), 7.49 (d, 2H, J=8.70 Hz), 7.86-7.97 (m, 2.5H), 8.15 (s, 0.5H), 9.71 (bs, 0.5H), 10.20 (s, 0.5H);
Anal. Calcd. for C₂₁H₂₂NO₈SF₃: C, 49.90; H, 4.38; N, 2.77. Found: C, 49.90; H, 4.35; N, 2.52.

### Example 3

### (1R)-1-((4R)-2,2-dimethyl-1,3-dioxolan-4-yl)-2-((4-(4-(trifluoromethoxy)phenoxy)phenyl)sulfonyl)ethyl(hydroxy)formamide

### Examples 3A and 3B

### (4R)-4-((1R)-1-(hydroxyamino)-2-((4-(4-trifluoromethoxy)phenoxy)phenyl)sulfonyl)ethyl)-2,2-dimethyl-1,3-dioxolane and

### (4S)-4-((1S)-1-(hydroxyamino)-2-((4-(4-(trifluoromethoxy)phenoxy)phenyl)sulfonyl)ethyl)-2,2-dimethyl-1,3-dioxolane

The desired product was prepared by substituting methyl (4S)-2,2-dimethyl-1,3-dioxolane-4-carboxylate for methyl (4R)-2,2-dimethyl-1,3-dioxolane-4-carboxylate in Examples 1A-1E.
MS (ESI) *m*/*z* 506 (M+H)⁺;
¹H NMR (300 MHz, DMSO-d₆): δ 1.21 (s, 1.5H), 1.23 (s, 1.5H), 1.26 (s, 1.5H), 1.30 (s, 1.5H), 3.3-3.4 (m, 1H), 3.60-3.75 (m, 2H), 3.9-4.1 (m, 2.5H), 4.5-4.6 (m, 0.5H), 7.2-7.3 (m, 4H), 7.48 (d, 2H, J=8.7 Hz), 7.81 (s, 0.5H), 7.85-7.95 (m, 2H), 8.13 (s, 0.5H), 9.63 (br s, 0.5H), 10.0 (br s, 0.5H);
Anal. Calcd. for C₂₁H₂₂F₃NO₈S: C, 49.90; H, 4.38; N, 2.77. Found: C, 49.90; H, 4.51; N, 2.66.

### Example 3C

### (1R)-1-((4R)-2,2-dimethyl-1,3-dioxolan-4-yl)-2-((4-(4-(trifluoromethoxy)phenoxy)phenyl)sulfonyl)ethyl(hydroxy)formamide

The desired product was prepared by substituting Example 3B for Example 1E in Example 2.

### Example 4

### (1S)-1-((4R)-2,2-dimethyl-1,3-dioxolan-4-yl)-2-((4-(4-(trifluoromethoxy)phenoxy)phenyl)sulfonyl)ethyl(hydroxy)formamide

The desired product was prepared by substituting Example 3A for Example 1D in Example 1F.
MS (ESI) *m*/*z* 506 (M+H)⁺;
¹H NMR (300 MHz, DMSO-d₆): δ 1.05 (s, 1.5H), 1.14 (s, 1.5H), 1.20 (s, 1.5H), 1.23 (s, 1.5H), 3.3-3.4 (m, 1H), 3.5-4.1 (m, 4.5H), 4.3-4.4 (m, 0.5H), 7.2-7.3 (m, 4H), 7.48 (d, 2H), 7.8-8.0 (m, 2.5H), 8.15 (s, 0.5H), 9.68 (br s, 0.5H), 10.10 (br s, 0.5H).

### Example 5

### 4-chloro-4'-(((2S)-2-((4S)-2,2-dimethyl-1,3-dioxolan-4-yl)-2-(formyl(hydroxy)amino)ethyl)sulfonyl)-1,1'-biphenyl

### Example 5A

### 4'-chloro(1,1'-biphenyl)-4-yl methylsulfone

The desired product was prepared by substituting 4-chlorophenylboronic acid for 4-trifluoromethylphenylboronic acid in Example 12A.

### Example 5B

### 4-chloro-4'-(((2S)-2-((4S)-2,2-dimethyl-1,3-dioxolan-4-yl)-2-(formyl(hydroxy)amino)ethyl)sulfonyl)-1,1'-biphenyl

The desired product was prepared by substituting Example 5A for 4-(4'-trifluoromethoxyphenoxy)phenyl methyl sulfone in Example 1.
MS (ESI) *m*/*z* 440 (M+H)⁺;
¹H NMR (DMSO-d₆): δ 1.20 (s, 1.5H), 1.22 (s, 1.5H), 1.26 (s, 1.5H), 2.30 (s, 1.5H), 3.32-3.40 (m, 1H), 3.62-3.78 (m, 2H), 3.93-4.15 (m, 2.5H), 4.64 (t, 0.5H, J=8.4 Hz), 7.58 (d, 2H, J=8.4 Hz), 7.77-7.83 (m, 2H), 7.89 (s, 0.5H), 7.93-8.02 (m, 4H), 8.13 (s, 0.5H), 9.62 (bs, 0.5H), 9.97 (bs, 0.5H);
Anal. Calcd. for C₂₀H₂₂NO₆SCl: C, 54.60; H, 5.04; N, 3.18. Found: C, 54.48; H, 5.30; N, 3.13.

### Example 6

### (1S)-1-((4S)-2,2-diethyl-1,3-dioxolan-4-yl)-2-((4-(4-(trifluoromethoxy)phenoxy)phenyl)sulfonyl)ethyl(hydroxy)formamide

### Example 6A

### (1RS)-1-((4R)-2,2-diethyl-1,3-dioxolan-4-yl)-2-((4-(4-(trifluoromethoxy)phenoxy)phenyl)sulfonyl)ethanol

A solution of Example 1A (1.0 g, 3.0 mmol) in THF (50 mL) at -78 °C was treated with n-butyllithium (2.5M in hexanes, 1.3 mL, 3.3 mmol) and stirred for 1.5 hours. The solution was added by cannula to a -78 °C solution of (R)-2,2-diethyl-1,3-dioxolane-4-carboxaldehyde (0.95 g, 6.0 mmol) (prepared according to the procedure described in Synthesis, 1992, p. 587) in THF (10 mL), stirred for 3 hours, treated with saturated NH₄Cl, and extracted with ethyl acetate. The combined extracts were washed with brine, dried (MgSO₄), filtered, and concentrated. The concentrate was purified first by flash column chromatography on silica gel with 3:1/hexanes:ethyl acetate to 3:2/hexanes:ethyl acetate, then by HPLC with 3:2/hexanes:ethyl acetate to provide the desired product as a mixture of diastereomers.

### Example 6B

### (1S)-1-((4S)-2,2-diethyl-1,3-dioxolan-4-yl)-2-((4-(4-(trifluoromethoxy)phenoxy)phenyl)sulfonyl)ethyl(hydroxy)formamide

The desired product was prepared by substituting Example 6A for Example 1B in Example 1C (omitting the sodium borohydride reduction), then substituting the resulting product for Example 1C in Examples 1D and 1F.
MS (ESI) *m*/*z* 534 (M+H)⁺;
¹H NMR (300 MHz, DMSO-d₆): δ 0.7-0.8 (m, 6H), 1.4-1.6 (m, 4H), 3.2-3.3 (m, 1H), 3.45-3.55 (m, 1H), 3.69 (dd, 1H, J=8.7,15.6 Hz), 3.95-4.15 (m, 2.5H), 4.5-4.6 (m, 0.5H), 7.2-7.3 (m, 4H), 7.47 (d, 2H, J=8.4 Hz), 7.81 (s, 0.5H), 7.85-7.95 (m, 2H), 8.14 (s, 0.5H), 9.66 (br s, 0.5H), 10.11 (br s, 0.5H);
Anal. Calcd. for C₂₃H₂₆NO₈SF₃: C, 51.77; H, 4.91; N, 2.62. Found: C, 51.98; H, 5.12; N, 2.63.

### Example 7

### 1,4,5-trideoxy-4-(formyl(hydroxy)amino)-2,3-O-(1-methylethylidene)-5-((4-(4-(trifluoromethoxy)phenoxy)phenyl)sulfonyl)-D-xylitol

The desired product was prepared by substituting methyl 3,4-isopropylidene-L-threonate for methyl (R)-2,2-dimethyl-1,3-dioxolane-4-carboxylate in Example 1.
MS (ESI) *m*/*z* 520 (M+H)⁺;
¹H NMR (300 MHz, DMSO-d₆): δ 1.2-1.3 (m, 9H), 3.3-3.5 (m, 2H), 3.6-3.9 (m, 3H), 4.1-4.2 (apparent t, 0.5H, J=5.0 Hz), 4.6-4.7 (apparent t, 0.5H, J=5.0 Hz), 7.2-7.3 (m, 4H), 7.48 (d, 2H, J=9.0 Hz), 7.85-8.00 (m, 2.5H), 8.15 (s, 0.5H), 9.69 (s, 0.5H), 9.95 (s, 0.5H); Anal. Calcd. for C₂₂H₂₄NO₈SF₃: C, 50.86; H, 4.65; N, 2.69. Found: C, 51.01; H, 4.38; N, 2.47.

### Example 8

### (1S)-1-((4S)-2,2-dimethyl-1,3-dioxolan-4-yl)-2-((4-(4-methoxyphenoxy)phenyl)sulfonyl)ethyl(hydroxy)formamide

The desired product was prepared by substituting 4-methoxyphenol for 4-trifluoromethoxyphenol in Example 1.
mp: 167.8-169 °C; (α)_{D}+4.4°(c 0.4, CH₃OH);
MS (ESI) *m*/*z* 452 (M+H)⁺;
¹H NMR (DMSO-d₆): δ 1.20-1.32 (m, 6H), 3.24-3.35 (m, 1H), 3.58-3.70 (m, 2H), 3.78 (s, 3H), 3.92-4.13 (m, 2.5H), 4.57 (t, 0.5H, J=8:1- Hz), 7:00-7.14 (m, 6H), 7.84 (dd, 2H, J=12.3,2.1 Hz), 7.89 (s, 0.5H), 8.13 (s, 0.5H), 9.64 (s, 0.5H), 10.02 (s, 0.5H);
Anal. Calcd. for C₂₁H₂₅NO₈S: C, 55.87; H, 5.58; N, 3.10. Found: C, 55.72; H, 5.59; N, 2.96.

### Example 9

### (1S)-2-((4-(4-chlorophenoxy)phenyl)sulfonyl)-1-((4S)-2,2-dimethyl-1,3-dioxolan-4-yl)ethyl(hydroxy)formamide

The desired product was prepared by substituting 4-chlorophenol for 4-trifluoromethoxyphenol in Example 1.
mp: 157-158 °C; (α)_{D}+2.2°(c 0.4, CH₃OH);
MS (ESI) *m*/*z* 456 (M+H)⁺;
¹H NMR (DMSO-d₆): δ 1.19-1.33 (m, 6H), 3.28-3.36 (m, 1H), 3.50-3.72 (m, 2H), 3.92-4.13 (m, 2.5H), 4.55 (t, 0.5H, J=8.1 Hz), 7.15-7.24 (m, 4H), 7.49-7.56 (m, 2H), 7.81 (s, 0.5H), 7.90 (t, 2H, J=9.3 Hz), 8.12 (s, 0.5H), 9.62 (s, 0.5H), 10.03 (s, 0.5H);
Anal. Calcd. for C₂₀H₂₂NO₇SCl: C, 52.69; H, 4.86; N, 3.07. Found: C, 52.67; H, 4.79; N, 2.87.

### Example 10

### (1S)-1-((4S)-2,2-dimethyl-1,3-dioxolan-4-yl)-2-((4-(4-(trifluoromethyl)phenoxy)phenyl)sulfonyl)ethyl(hydroxy)formamide

The desired product was prepared by substituting 4-trifluoromethylphenol for 4-trifluoromethoxyphenol in Example 1.
mp: 141-143 °C; (α)_{D}+2.0°(c 0.1, CH₃OH);
MS (APCI) *m*/*z* 490 (M+H)⁺;
¹H NMR (DMSO-d₆): δ 1.20-1.33 (m, 6H), 3.35-3.41 (m, 1H), 3.62-3.77 (m, 2H), 3.95-4.15 (m, 2.5H), 4.57 (t, 0.5H, J=8 Hz), 7.27-7.35 (m, 4H), 7.77-7.85 (m, 2.5H), 7.91-7.99 (m, 2H), 8.13 (s, 0.5H), 9.50-9.85 (m, 1H); Anal. Calcd. for C₂₁H₂₂F₃NO₇S: C, 51.53; H, 4.53; N, 2.86 Found: C, 51.60; H, 4.61; N, 2.88.

### Example 11

### (1S)-1-((4S)-2,2-dimethyl-1,3-dioxolan-4-yl)-2-((4-(4-methylphenoxy)phenyl)sulfonyl)ethyl(hydroxy)formamide

The desired product was prepared by substituting 4-methylphenol for 4-trifluoromethoxyphenol in Example 1.
mp: 156-158 °C; (α)_{D}+5.0°(c 0.2, CH₃OH);
MS (APCI) *m*/*z* 436 (M+H)⁺;
¹H NMR (DMSO-d₆): δ 1.17-1.36 (m, 6H), 3.21-3.31 (m, 1H), 3.34 (s, 3H), 3.58-3.73 (m, 2H), 3.91-4.16 (m, 2.5H), 4.57-4.64 (m, 0.5H), 6.97-7.06 (m, 2H), 7.10 (d, 2H, J=9 Hz), 7.26 (d, 2H, J=9 Hz), 7.78-7.93 (m, 2.5H), 8.13 (s, 0.5H), 9.41-10.12 (bs, 1H);
Anal. Calcd. for C₂₁H₂₅NO₇S: C, 57.92; H, 5.79; N, 3.22;S,7.36. Found: C,57.63; H, 5.81; N,3.11;S,7.21.

### Example 12

### 4-(((2S)-2-((4S)-2,2-dimethyl-1,3-dioxolan-4-yl)-2-(formyl(hydroxy)amino)ethyl)sulfonyl)-4'-(trifluoromethyl)-1,1'-biphenyl

### Example 12A

### methyl 4'-(trifluoromethyl)(1,1'-biphenyl)-4-ylsulfone

A solution of 4-(trifluoromethyl)phenylboronic acid (12.0g, 62 mmol) and 4-bromophenyl methyl sulfone (14.93g, 62 mmol) in DMF (200 mL) was treated with Cs₂CO₃ (61g, 187 mmol) and PdCl₂(dppf)₂ (1.5g), heated to 60 °C, stirred for 3 hours, cooled to room temperature, and stirred for 16 hours. The mixture was partitioned between ethyl acetate and water and the organic phase was washed sequentially with 1:1/brine:water and brine, dried (Na₂SO₄), filtered, and concentrated. The concentrate was recrystallized from ethyl acetate/hexanes to provide the desired product.
MS (APCI) *m*/*z* 318 (M+NH₄)⁺.

### Example 12B

### 4-(((2S)-2-((4S)-2,2-dimethyl-1,3-dioxolan-4-yl)-2-(formyl(hydroxy)amino)ethyl)sulfonyl)-4'-(trifluoromethyl)-1,1'-biphenyl

The desired product was prepared by substituting Example 12A for Example 1A in Examples 1B-1F.
mp: 204-205 °C; (α)_{D} +6.0°(c 0.1, CH₃OH);
MS (ESI) *m*/*z* 474 (M+H)⁺;
¹H NMR (DMSO-d₆): δ 1.13-1.42 (m, 6H), 3.35-3.48 (m, 1H), 3.62-3.85 (m, 2H), 3.93-4.24 (m, 2.5H), 4.61-4.76 (m, 0.5H), 7.79-8.27 (m, 9H), 9.79-10.20 (m, 1H);
Anal. Calcd. for C₂₁H₂₂F₃NO₆S: C, 53.27; H, 4.68; N, 2.96; S, 6.77; F, 12.04. Found: C, 53.09; H, 4.74; N, 2.89; S, 6.79; F, 12.21.

### Example 13

### (1S)-1-((4S)-1,3-dioxolan-4-yl)-2-((4-(4-(trifluoromethoxy)phenoxy)phenyl)sulfonyl)ethyl(hydroxy)formamide

### Example 13A

### (2R,3E)-4-((4-(4-(trifluoromethoxy)phenoxy)phenyl)sulfonyl)-3-butene-1,2-diol

A solution of Example 1C (760 mg, 0.95 mmol) in THF (15mL) was treated with 3N HCl (3 mL), heated to 45 °C, stirred for 1.5 hours, cooled to room temperature, and extracted with diethyl ether. The combined extracts were washed with brine, dried (Na₂SO₄), filtered and concentrated to provide the desired product.
MS(DCI) *m*/*z* 422 (M+NH₄)⁺.

### Example 13B

### (4S)-4-((E)-2-((4-(4-(trifluoromethoxy)phenoxy)phenyl)sulfonyl)ethenyl)-1,3-dioxolane

A solution of Example 13A (970 mg) in DMSO (12 mL) at 65 °C was treated with POCl₃, stirred for 2.5 hours, and partitioned between diethyl ether and water. The organic phase was washed with brine, dried (Na₂SO₄), filtered, and concentrated. The concentrate was purified by flash column chromatography on silica gel using 9:1/dichloromethane:hexanes, then dichloromethane, then 9:1/dichloromethane:ethyl acetate to provide the desired product.
MS(DCI) *m*/*z* 434 (M+NH₄)⁺.

### Example 13C

### (1S)-1-((4S)1,3-dioxolan-4-yl)-2-((4-(4-(trifluoromethoxy)phenoxy)phenyl)sulfonyl)ethyl(hydroxy)formamide

The desired product was prepared by substituting Example 13B for Example 1B in Examples 1D and 1F.
MS (ESI) *m*/*z* 476 (M-H)⁻;
¹H NMR (DMSO-d₆): δ 3.53-4.16 (m, 5.5H), 4.51-4.63 (m, 0.5H), 4.74 (d, 1H, J=12 Hz), 4.84 (s, 0.5H), 4.95 (s, 0.5H), 7.23 (d, 2H, J=9 Hz), 7.28 (d, 2H, J=9 Hz), 7.48 (d, 2H, J=9 Hz), 7.83 (s, 0.5H), 7.94 (dd, 2H, J=9,8.8 Hz), 8.17 (s, 0.5H), 9.15 (s, 0.5H), 10.03 (s, 0.5H);
Anal. Calcd. for C₁₉H₁₈NO₈SF₃: C, 47.80; H, 3.80; N, 2.93. Found: C, 47.55; H, 3.76; N, 2.82.

### Example 14

### 4-(((2S)-2-((4S)-2,2-dimethyl-1,3-dioxolan-4-yl)-2-(formyl(hydroxy)amino)ethyl)sulfonyl)-4'-(2-methoxyethoxy)-1,1'-biphenyl

### Example 14A

### 4-(2-methoxyethoxy)-4'-(methylsulfanyl)-1,1'-biphenyl

The desired product was prepared by substituting 4-(methylsulfanyl)phenylboronic acid and 1-bromo-4-(2-methoxyethoxy)benzene for 4-(trifluoromethyl)phenylboronic acid and 4-bromophenyl methyl sulfone, respectively, in Example 12A.

### Example 14B

### 4'-(2-methoxyethoxy)(1,1'-biphenyl)-4-yl methylsulfone

A suspension of Example 14A (2.8g, 10.2 mmol) in 2:1/methanol:water (100 mL) at 0 °C was treated with NaHCO₃ ( 2.14g, 25.3 mmol) and oxone ( 15.7g, 25.3 mmol), stirred for 1 hour, warmed to room temperature, and stirred for 48 hours. The mixture was partitioned between water and dichloromethane and the aqueous phase was extracted with dichloromethane. The combined extracts were washed with brine, dried (Na₂SO₄), filtered, and concentrated. The concentrate was purified by flash column chromatography on silica gel with 1:1/hexanes:ethyl acetate to provide the desired product.
MS (ESI) *m*/*z* 307 (M+H)⁺.

### Example 14C

### 4-(((2S)-2-((4S)-2,2-dimethyl-1,3-dioxolan-4-yl)-2-(formyl(hydroxy)amino)ethyl)sulfonyl)-4'-(2-methoxyethoxy)-1,1'-biphenyl

The desired product was prepared by substituting Example 14B for Example 1A in Examples 1B-1D and 1F.
MS (ESI) *m*/*z* 478 (M-H)⁻;
¹H NMR (DMSO-d₆): δ 1.21 (d, 3H, J=9 Hz), 1.25-1.35 (m, 3H), 3.28-3.42 (m, 4H), 3.46-3.57 (m, 1H), 3.10-3.26 (m, 3H), 3.86-4.20 (m, 4H), 4.29-4.45 (m, 0.5H), 4.57-4.68 (m, 0.5H), 7.08 (d, 2H, J=9 Hz), 7.68-7.77 (m, 2H), 7.83-7.97 (m, 4.5H), 8.14 (s, 0.5H), 9.62 (s, 0.5H), 9.98 (s, 0.5H);
Anal. Calcd. for C₂₃H₂₉NO₈S: C, 57.60; H, 6.09; N, 2.92. Found: C, 57.61; H, 6.10; N, 2.92.

### Example 15

### (1S)-1-((4S)-2,2-dimethyl-1,3-dioxolan-4-yl)-2-((4-(4-(2-methoxyethoxy)phenoxy)phenyl)sulfonyl)ethyl(hydroxy)formamide

### Example 15A

### 1-(benzyloxy)-4-(2-methoxyethoxy)benzene

A solution of 4-(benzyloxy)phenol (6.1g, 30.5 mmol), 2-methoxyethanol (2.4 mL, 30.5 mmol) and triphenylphosphine (8.78g, 30.5 mmol) in THF (150 mL) at 0 °C was treated with diethylazodicarboxylate (5.3 mL, 33.5 mmol), stirred for 10 minutes, warmed to room temperature, and stirred for 12 hours. The mixture was diluted with ethyl acetate, washed with 2N NaOH and brine, dried (Na₂SO₄), filtered, and concentrated. The concentrate was purified by flash column chromatography on silica gel with 9:1/hexanes:ethyl acetate to provide the desired product.

### Example 15B

### 4-(2-methoxyethoxy)-phenol

A solution of Example 15A (4.8 g, 18.6 mmol) in methanol (48 mL) was treated with 20% Pd(OH)₂ on carbon (0.48 g) and stirred under 60 psi of hydrogen at 50 °C for 1 hour. The mixture was filtered and the filtrate was concentrated to provide the desired product.
MS (DCI) *m*/*z* 186 (M+NH₄)⁺.

### Example 15C

### (1S)-1-((4S)-2,2-dimethyl-1,3-dioxolan-4-yl)-2-((4-(4-(2-methoxyethoxy)phenoxy)phenyl)sulfonyl)ethyl(hydroxy)formamide

The desired product was prepared by substituting Example 15B for 4-trifluoromethoxyphenol in Example 1.
¹H NMR (DMSO-d₆): δ 1.22 (d, 3H, J=9 Hz), 1.28 (d, 3H, J=12 Hz), 3.22-3.35 (m, 3H), 3.57-3.60 (m, 4H), 3.93-4.15 (m, 5.5H), 4.52-4.63 (m, 0.5H), 7.0-7.13 (m, 6H), 7.83 (d, 0.5H, J=3 Hz), 7.87 (d, 2H, J=10 Hz), 8.12 (s, 0.5H), 9.63 (s, 0.5H), 9.98 (s, 0.5H);
Anal. Calcd. for C₂₃H₂₉O₉SN: C, 55.74; H, 5.89; N, 2.82. Found: C, 55.65; H, 5.82; N, 2.79.

### Example 16

### 1,2,4-trideoxy-2-(formyl(hydroxy)amino)-4,4-dimethyl-3,5-O-(1-methylethylidene)-1-((4-(4-(trifluoromethoxy)phenoxy)phenyl)sulfonyl)-D-threo-pentitol

### Example 16A

### (3R)-3-((tert-butyl(dimethyl)silyl)oxy)-5-hydroxy-4,4-dimethyl-1-((4-(4-(trifluoromethoxy)phenoxy)phenyl)sulfonyl)-2-pentanone

The desired product was prepared by substituting (3R)-3-((tert-butyl(dimethyl)silyl)oxy)-4,4-dimethyldihydro-2(3H)-furanone and methyl 4-(4-(trifluoromethoxy)phenoxy)phenyl sulfone for methyl (4R)-2,2-dimethyl-1,3-dioxolane-4-carboxylate and 4-(4'-trifluoromethoxyphenoxy)phenyl methyl sulfone, respectively, in Example 1B.

### Example 16B

### (3R)-3,5-dihydroxy-1-((4-(4-(trifluoromethoxy)phenoxy)phenyl)sulfonyl)-2-pentanone

A solution of Example 16A (1.7 g, 2.9 mmol) in THF (10 mL) at 0 °C was treated with tetrabutylammonium fluoride (1M in THF, 8.7 mL, 8.7 mmol), stirred for 1 hour, warmed to room temperature, stirred for 3 hours, and partitioned between ethyl acetate and brine. The organic phase was washed with brine, dried (MgSO₄), filtered, and concentrated. The concentrate was purified by flash column chromatography on silica gel with 65:35/hexanes:ethyl acetate to provide the desired product.

### Example 16C

### 1-((4R)-2,2-dimethyl-1,3-dioxan-4-yl)-2-((4-(4-(trifluoromethoxy)phenoxy)phenyl)sulfonyl)ethanone

A solution of Example 16B (1.1g, 2.4 mmol) and 2,2-propanediol (350 mL) in DMF (10 mL) at room temperature was treated with catalytic camphorsulfonic acid, stirred for 15 hours, and partitioned between water and ethyl acetate. The organic phase was washed with brine, dried (MgSO₄), filtered, and concentrated. The concentrate was purified by flash column chromatography on silica gel with 3:1/hexanes:ethyl acetate to provide the desired product.

### Example 16D

### 1,2,4-trideoxy-2-(formyl(hydroxy)amino)-4,4-dimethyl-3,5-O-(1-methylethylidene)-1-((4-(4-(trifluoromethoxy)phenoxy)phenyl)sulfonyl)-D-threo-pentitol

The desired product was prepared by substituting Example 16C for Example 1B in Examples 1C, 1D, and 1F.
MS (ESI) *m*/*z* 546 (M-H)⁻;
¹H NMR (DMSO-d₆): δ 0.64-0.83 (m, 3H), 0.90 (s, 3H), 1.25-1.33 (m, 6H), 3.03-3.14 (m, 1H), 3.33-3.54 (m, 2H), 3.62-3.72 (m, 1H), 3.73-3.85 (m, 1H), 3.92-4.05 (m, 0.5H), 4.69-4.78 (m, 0.5H), 7.19-7.20 (m, 4H), 7.48 (d, 2H, J=9 Hz), 7.76 (s, 0.5H), 7.88-7.97 (m, 2H), 8.05 (s, 0.5H), 9.28 (s, 1H);
Anal. Calcd. for C₂₄H₂₈NO₈SF₃: C, 52.64; H, 5.15; N, 2.55. Found: C, 52.83; H, 5.30; N, 2.30.

### Example 17

### hydroxy((1S)-1-((2R)-tetrahydro-2-furanyl)-2-((4-(4-(trifluoromethoxy)phenoxy)phenyl)sulfonyl)ethyl)formamide

### Examples 17A and 17B

### (2R)-2-((1S)-1-(hydroxyamino)-2-((4-(4-(trifluoromethyl)phenoxy)phenyl)sulfonyl)ethyl)tetrahydrofuran and

### (2R)-2-((1R)-1-(hydroxyamino)-2-((4-(4-(trifluoromethyl)phenoxy)phenyl)sulfonyl)ethyl)tetrahydrofuran

The desired product was prepared by substituting methyl (2R)-tetrahydro-2-furancarboxylate for methyl (4R)-2,2-dimethyl-1,3-dioxolane-4-carboxylate in Examples 1A-1E.

### Example 17C

### hydroxy((1S)-1-((2R)-tetrahydro-2-furanyl)-2-((4-(4-(trifluoromethoxy)phenoxy)phenyl)sulfonyl)ethyl)formamide

The desired product was prepared by substituting Example 17A for Example 1D in Example 1F.
mp: 121-122 °C; (α)_{D} = -2.5° (c 1.08, CH₃OH);
MS (ESI, +Q1MS) *m*/*z* 476 (M+H)⁺;
¹H NMR (500MHz, DMSO-d₆): δ 1.37-1.45 (m, 1H), 1.71-1.82 (m, 2H), 1.88-1.99 (m, 1H), 3.28 (m, 0.6H), 3.56-3.73 (m, 4H), 3.81-3.91 (m, 2H), 4.46 (t, 0.4H, J=10.0 Hz), 7.22 (d, 2H, J=9.0 Hz), 7.25-7.29 (m, 2H), 7.46 (d, 2H, J=8.5 Hz), 7.77 (s, 0.6H), 7.90 (d, 2H, J=9.0 Hz), 7.93 (d, 2H, J=8.5 Hz), 8.12 (S, 0.4H), 9.45 (s, br, 0.6H), 9.82 (s, br, 0.4H); Anal. Calcd. for C₂₀H₂₀F₃NO₇S: C, 50.52; H, 4.24; N, 2.95. Found: C, 50.69; H, 4.47; N, 2.89.

### Example 18

### hydroxy((1R)-1-((2R)-tetrahydro-2-furanyl)-2-((4-(4-(trifluoromethoxy)phenoxy)phenyl)sulfonyl)ethyl)formamide

The desired product was prepared by substituting Example 17B for Example 1E in Example 2.
mp: 142.5-143.5 °C; (α)_{D} = -23.8°(α)_{D}+2.0°(c 0.98. CH₃OH);
MS (ESI, +Q1MS) *m*/*z* 476 (M+H)⁺;
¹H NMR (400MHz, DMSO-d6): δ 1.47-3.93 (m, 4H), 3.38-3.93 (m, 5.5H), 4.36 (t, 0.5H, J=8.8 Hz), 7.20-7.30 (m, 4H), 7.46 (d, 2H, J=8.88 Hz), 7.87-7.92 (m, 2.5H), 8.11 (s, 0.5H), 9.63 (s, br, 0.5H), 10.05 (s, br, 0.5H);
Anal. Calcd. for: C₂₀H₂₀F₃NO₇S: C, 50.52; H, 4.24; N, 2.95. Found: C, 50.76; H, 4.34; N, 2.77.

### Example 19

### hydroxy((1S)-1-((2R)-1-(methylsulfonyl)pyrrolidinyl)-2-((4-(4-(trifluoromethoxy)phenoxy)phenyl)sulfonyl)ethyl)formamide

### Example 19A

### methyl (2R)-1-(methylsulfonyl)-2-pyrrolidinecarboxylate

A 0 °C solution of D-proline methyl ester hydrochloride (4.5g, 27 mmol) (prepared according to the procedure described in Synthesis, 195,p. 772) in dichloromethane (200 mL) at room temperature was treated with triethylamine (11.3 mL, 81 mmol) and methylsulfonyl chloride (3.13 mL, 40 mmol), then stirred for 4 hours. The reaction was partitioned between saturated NH₄Cl and dichloromethane, and the organic phase was washed sequentially with saturated NaHCO₃ and brine, dried (Na₂SO₄), filtered, and concentrated to provide the desired product.
MS (DCI) *m*/*z* 225 (M+NH₄)⁺.

### Example 19B

### hydroxy((1S)-1-((2R)-1-(methylsulfonyl)pyrrolidinyl)-2-((4-(4-(trifluoromethoxy)phenoxy)phenyl)sulfonyl)ethyl)formamide

The desired product was prepared by substituting Example 19A for methyl (R)-2,2-dimethyl-1,3-dioxolane-4-carboxylate in Example 1.
MS (ESI) m/e 553 (M+H)⁺, 570 (M+NH₄)⁺, 551 (M-H)⁻; (α)_{D:}-12.33°C, (CHCl3, c 0.3);
¹H NMR (300 MHz, DMSO-d₆): δ 1.65-1.78 (m, 3H), 1.93-2.08 (m, 1H), 2.85 (s, 0.4H), 2.88 (s, 0.6H), 3.12-3.45 (m, 3H), 3.71-3.79 (m, 0.6H), 4.23-4.28 (m, 0.4H), 7.19-7.29 (m, 4H), 7.43-7.46 (d, 2H, J=8.7 Hz), 7.80 (s, 0.6H), 7.86-7.92 (m, 2H), 8.17 (s, 0.4H), 9.48 (s, 0.6H), 9.71 (s, 0.4H).
High resolution MS (FAB) Calc. *m*/*z* for (M+H)⁺ 553.0926, observed *m*/*z* 553.0930.

### Example 20

### hydroxy((1RS)-1-((4S)-2-oxo-1,3-oxazolidin-4-yl)-2-((4-(4-(trifluoromethoxy)phenoxy)phenyl)sulfony)ethyl)formamide

The desired product was prepared as a mixture of diastereomers by substituting methyl (4R)-2-oxo-1,3-oxazolidine-4-carboxylate (prepared according to the procedure described in Tet. Lett. 1994, p. 2397) for methyl (R)-2,2-dimethyl-1,3-dioxolane-4-carboxylate in Example 1.
MS (ESI +Q1MS) *m*/*z* 508 (M+NH₄)⁺;
¹H NMR (400MHz, DMSO-d₆): δ 3.42-3.60 (m, 1H), 3.68-4.00 (m, 3H), 4.05-4.60 (m, 2H), 7.20-7.32 (m, 4H), 7.47 (d, 2H, J=6.6 Hz), 7.80-7.96 (M, 3.5H), 8.15 (S, 0.5H), 9.53 (S, br, 0.25H), 9.71 (S, br, 0.25H), 9.97 (s, br, 0.25H), 10.12 (s, br, 0.25H);
Anal. Calcd. for C₁₉H₁₇F₃N₂O₈S: C, 46.53; H, 3.49; N, 5.71. Found: C, 46.26; H, 3.43; N, 5.57.

### Example 21

### hydroxy((1RS)-1-((4S)-3-methyl-2-oxo-1,3-oxazolidin-4-yl)-2-((4-(4-(trifluoromethoxy)phenoxy)phenyl)sulfonyl)ethyl)formamide

The desired product was prepared as a mixture of diastereomers by substituting methyl (4R)-3-methyl-2-oxo-1,3-oxazolidine-4-carboxylate for methyl (R)-2,2-dimethyl-1,3-dioxolane-4-carboxylate in Example 1.
MS (ESI, Q+1MS) *m*/*z* 505 (M+H)⁺, 522 (M+NH₄)⁺;
¹H NMR (400MHz, DMSO-d₆): δ 2.69 (s, 1.5H), 2.76 (s, 1.5H), 3.54-3.65 (m, 1H), 3.79-3.94 (m, 2H), 4.21 (t, 1H, J=8.8 Hz), 4.27-4.35 (m, 1H), 4.49 (m, 0.5H), 4.89 (m, 0.5H), 7.22-7.32 (m, 4H), 7.47 (d, 2H), 7.90-7.98 (m, 2.5H), 8.17 (s, 0.5H), 9.88 (s, br, 0.5H), 10.20 (s, br, 0.5H);
Anal. Calcd. for C₂₀H₁₉F₃N₂O₈S: C, 47.62; H, 3.80; N, 5.55. Found: C, 47.95; H, 4.03; N, 5.34.

### Example 22

### 1,2-dideoxy-2-(formyl(hydroxy)amino)-3,4-O-(1-methylethylidene)-1-((4-(4-(trifluoromethoxy)phenoxy)phenyl)sulfonyl)-L-threo-pentitol

The desired product was prepared by substituting (2S,3S)-2,3-O-isopropylidine-2,3,4-trihydroxybutanal tert-butyldimethylsilyl ether (prepared by the procedure described n J. Org. Chem. 1993,v. 58, p. 5153) for (R)-2,2-diethyl-1,3-dioxolane-4-carboxaldehyde n Example 6A, then substituting the resulting product for Example 6A in Example 6B and Example 16B.
MS (ESI) *m*/*z* 536 (M+H)⁺, 553 (M+NH₄)⁺;
¹H NMR (400 MHz, DMSO-d6): δ 9.96 (s, 0.5H), 9.62 (s, 0.5H), 8.14 (s, 0.5H), 7.94-7.88 m, 2H), 7.81 (s, 0.5H), 7.48-7.45 (m, 2H), 7.29-7.20 (m, 4H), 5.18-5.12 (m, 1H), 4.72-4.62 (m, 1H), 4.10-3.92 (m, 2H), 3.80-3.62 (m, 2H), 3.60-3.30 (m, 4H), 1.29-1.22 (m, 6H).

It will be evident to one skilled in the art that the instant invention is not limited to the forgoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning and range of equivalency of the claims and therefore intended to be embraced therein.

## Claims

1. A compound of formula (I) 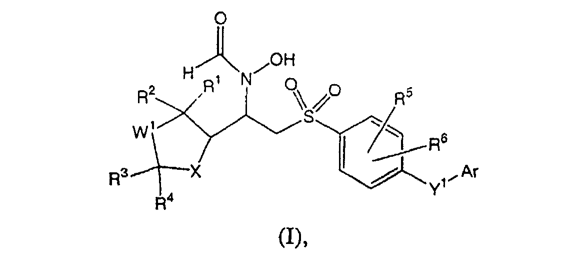 or a pharmaceutically acceptable salt or prodrug thereof, wherein
**W**^{**1**} is selected from the group consisting of
(1) -O-,
(2) -CH₂O-,
and
(3) -CH₂-;
wherein each group is drawn with its left-hand end being the end which attaches to the carbon containing R¹ and R², and its right-hand end being the end which attaches to the carbon containing R³ and R⁴;
**X** is selected from the group consisting of
(1) -O-,
and
(2) -N(R⁷)-, wherein R⁷ is selected from the group consisting of
(a) hydrogen,
(b) alkyl,
(c) -SO₂-alkyl,
and
(d) alkanoyl;
**R**^{**1**} and **R**^{**2**} are independently selected from the group consisting of
(1) hydrogen,
(2) alkyl,
and
(3) hydroxyalkyl;
**R**^{**3**} and **R**^{**4**} are independently selected from the group consisting of
(1) hydrogen,
and
(2) alkyl,
or
**R**^{**3**} and **R**^{**4**} taken together are oxo,
or
**R**^{**3**} and **R**^{**4**}, taken together with the carbon atom to which they are attached, form a cycloalkyl ring;
**R**^{**5**} and **R**^{**6**} are independently selected from the group consisting of
(1) hydrogen,
(2) alkyl,
(3) perfluoroalkyl,
(4) halo,
(5) haloalkyl,
(6) alkoxy,
(7) hydroxy,
(8) hydroxyalkyl,
(9) alkoxyalkyl,
and
(10) nitro;
**Y**^{**1**} is selected from the group consisting of
(1) a covalent bond,
(2) -O-,
(3) alkylene of two to four carbon atoms,
(4) piperidineneyl,
(5) alkenylene of two carbon atoms,
(6) alkynylene of two carbon atoms,
(7) -SO₂-,
and
(8)-C(O)-;
**Ar** is selected from the group consisting of
(1) phenyl,
(2) pyridyl,
(3) pyrazinyl,
(4) pyridazinyl,
(5) furyl,
(6) thienyl,
(7) isoxazolyl,
(8) oxazolyl,
(9) thiazolyl,
and
(10) isothiazolyl,
wherein (1)-(10) can be optionally substituted with one, two, or three substituents independently selected from the group consisting of
(a) alkyl,
(b) alkoxy, wherein the alkoxy can be optionally substituted with alkoxy,
(c) -(alkylene)-CO₂R₈, wherein R₈ is either hydrogen or alkyl,
(d) -(alkylene)-NR₉R₁₀, wherein R₉ and R₁₀ are independently selected from the group consisting of
(i) alkyl,
(ii) phenyl,
and
(iii) phenylalkyl,
wherein for (ii) and (iii), the phenyl and the phenyl part of the phenylalkyl can be optionally substituted with one or two substituents independently selected from the group consisting of halo and alkoxy,
(e) alkoxyalkyl,
(f) cyano,
(g) cyanoalkyl,
(h) halo,
(i) haloalkyl,
(j) hydroxy,
(k) hydroxyalkyl,
(l) thioalkoxy,
(m) thioalkoxyalkyl,
(n) phenylalkoxy,
(o) phenoxy,
(p) phenoxyalkyl,
(q) (heterocycle)oxy,
(r) (heterocycle)oxyalkyl,
(s) perfluoroalkyl,
(t) perfluoroalkoxy,
(u) sulfinylalkyl,
(v) sulfonylalkyl,
(w) 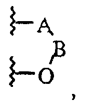 wherein A is selected from the group consisting of -CH₂-, -CH₂O- and -O-, and B is selected from the group consisting of -C(O)- and -(C(R")₂)ᵥ-, wherein R" is either hydrogen or alkyl, and v is 1-3,
and
(x) -N(R⁸)SO₂R¹¹, wherein R¹¹ is selected from the group consisting of
(i) hydrogen,
(ii) alkyl,
and
(iii) -N(R⁸)(R¹²), wherein R¹² is hydrogen or alkyl,
wherein for (q) and (r), the heterocycle part of (heterocycle)oxy, and (heterocycle)oxyalkyl are selected from the group consisting of
(i) pyridyl,
(ii) pyrazinyl,
(iii) pyridazinyl,
(iv) furyl,
(v) thienyl,
(vi) isoxazolyl,
(vii) oxazolyl,
(viii) thiazoloyl,
and
(ix) isothiazolyl,
and wherein for (q) and (r), the heterocycle part of the (heterocycle)oxy and the (heterocycle)oxyalkyl can be optionally substituted with one or two substituents independently selected from the group consisting of
(i) alkyl,
(ii) alkoxy,
(iii) perfluoroalkyl,
(iv) halo,
(v) cyano,
(vi) cyanoalkyl,
(vii) haloalkyl,
and
(viii) alkanoyl,
and
wherein for (o) and (p), the phenyl part of the phenoxy and the phenoxyalkyl can be optionally substituted with one or two substituents independently selected from the group consisting of
(i) alkyl,
(ii) alkoxy,
(iii) perfluoroalkyl,
(iv) halo,
(v) cyano,
(vi) cyanoalkyl,
(vii) haloalkyl,
and
(viii) alkanoyl.

2. A compound according to Claim 1, wherein Ar is phenyl.

3. A compound according to Claim 2, wherein Y¹ is -O-.

4. A compound according to Claim 3, wherein W¹ is -O-.

5. A compound according to Claim 4, wherein X is -O-.

6. A compound according to Claim 5 selected from the group consisting of (1S)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4R)-2,2-dimethyl-1,3-dioxolan-4-yl)-2-((4-(4-(trifluoromethoxy)phenoxy)phenyl)sulfonyl)ethyl(hydroxy)formamide, (1S)-1-((4R)-2,2-dimethyl-1,3-dioxolan-4-yl)-2-((4-(4-(trifluoromethoxy)phenoxy)phenyl)sulfonyl)ethyl(hydroxy)formamide, (1S)-1-((4S)-2,2-diethyl-1,3-dioxolan-4-yl)-2-((4-(4-(trifluoromethoxy)phenoxy)phenyl)sulfonyl)ethyl(hydroxy)formamide, 1,4,5-trideoxy-4-(formyl(hydroxy)amino)-2,3-O-(1-methylethylidene)-5-((4-(4-(trifluoromethoxy)phenoxy)phenyl)sulfonyl)-D-xylitol, (1S)-1-((4S)-2,2-dimethyl-1,3-dioxolan-4-yl)-2-((4-(4-methoxyphenoxy)phenyl)sulfonyl)ethyl(hydroxy)formamide, (1S)-2-((4-(4-chlorophenoxy)phenyl)sulfonyl)-1-((4S)-2,2-dimethyl-1,3-dioxolan-4-yl)ethyl(hydroxy)formamide, (1S)-1-((4S)-2,2-dimethyl-1,3-dioxolan-4-yl)-2-((4-(4-(trifluoromethyl)phenoxy)phenyl)sulfonyl)ethyl(hydroxy)formamide, (1S)-1-((4S)-2,2-dimethyl-1,3-dioxolan-4-yl)-2-((4-(4-methylphenoxy)phenyl)sulfonyl)ethyl(hydroxy)formamide, (1S)-1-((4S)-1,3-dioxolan-4-yl)-2-((4-(4-(trifluoromethoxy)phenoxy)phenyl)sulfonyl)ethyl(hydroxy)formamide, (1S)-1-((4S)-2,2-dimethyl-1,3-dioxolan-4-yl)-2-((4-(4-(2-methoxyethoxy)phenoxy)phenyl)sulfonyl)ethyl(hydroxy)formamide, and 1,2-dideoxy-2-(formyl(hydroxy)amino)-3,4-O-(1-methylethylidene)-1-((4-(4-(trifluoromethoxy)phenoxy)phenyl)sulfonyl)-L-threo-pentitol.

7. A compound according to Claim 4, wherein X is -N(R⁷)-.

8. A compound according to Claim 7 selected from the group consisting of hydroxyl((1RS)-1-((4S)-2-oxo-1,3-oxazolidin-4-yl)-2-((4-(4-(trifluoromethoxy)phenoxy)phenyl)sulfonyl)ethyl)formamide and hydroxy(( 1RS)-1-((4S)-3-methyl-2-oxo-1,3-oxazolidin-4-yl)-2-((4-(4-(trifluoromethoxy)phenoxy)phenyl)sulfonyl)ethyl)formamide.

9. A compound according to Claim 3, wherein W¹ is -CH₂-.

10. A compound according to Claim 9 wherein X is -O-.

11. A compound according to Claim 10 selected from the group consisting of hydroxy((1S)-1-((2R)-tetrahydro-2-furanyl)-2-((4-(4-(trifluoromethoxy)phenoxy)phenyl)sulfonyl)ethyl)formamide and hydroxy((1R)-1-((2R)-tetrahydro-2-furanyl)-2-((4-(4-(trifluoromethoxy)phenoxy)phenyl)sulfonyl)ethyl)formamide.

12. A compound according to Claim 9, wherein X is -N(R⁷)-.

13. A compound according to Claim 12 which is hydroxy((1S)-1-((2R)-1-(methylsulfonyl)pyrrolidinyl)-2-((4-(4-(trifluoromethoxy)phenoxy)phenyl)sulfonyl)ethyl)formamide.

14. A compound according to Claim 3, wherein W is -CH₂O-.

15. A compound according to Claim 14 which is 1,2,4-trideoxy-2-(formyl(hydroxy)amino)-4,4-dimethyl-3,5-O-(1-methylethylidene)-1-((4-(4-(trifluoromethoxy)phenoxy)phenyl)sulfonyl)-D-threo-pentitol.

16. A compound according to Claim 2, wherein Y is a covalent bond, W¹ is -O-, and X is -O-.

17. A compound according to Claim 16 consisting of 4-chloro-4'-(((2S)-2-((4S)-2,2-dimethyl-1,3-dioxolan-4-yl)-2-(formyl(hydroxy)amino)ethyl)sulfonyl)-1,1'-biphenyl, 4-(((2S)-2-((4S)-2,2-dimethyl-1,3-dioxolan-4-yl)-2-5 (formyl(hydroxy)amino)ethyl)sulfonyl)-4'-(trifluoromethyl)-1,1'-biphenyl, and 4-(((2S)-2-((4S)-2,2-dimethyl-1,3-dioxolan-4-yl)-2-(formyl(hydroxy)amino)ethyl)sulfonyl)-4'-(2-methoxyethoxy)-1,1'-biphenyl.

18. Use of a compound of Claim 1 for manufacturing a medicament for inhibiting matrix metalloproteinase in a mammal in recognized need of such treatment.

19. A compound according to claim 1 selected from the group consisting of (1S)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4S)-2,2-dimethyl-1,3-dioxolan-4-yl)-2-((4-(4-(trifluoromethoxy)phenoxy)phenyl)sulfonyl)ethyl(hydroxy)formamide, (1R)-1-((4R)-2,2-dimethyl-1,3-dioxolan-4-yl)-2-((4-(4-(trifluoromethoxy)phenoxy)phenyl)sulfonyl)ethyl(hydroxy)formamide, (1S)-1-((4R)-2,2-dimethyl-1,3-dioxolan-4-yl)-2-((4-(4-(trifluoromethoxy)phenoxy)phenyl)sulfonyl)ethyl(hydroxy)formamide, 4-chloro-4'-(((2S)-2-((4S)-2,2-dimethyl-1,3-dioxolan-4-yl)-2-(formyl(hydroxy)amino)ethyl)sulfonyl)-1,1'-biphenyl, (1S)-1-((4S)-2,2-diethyl-1,3-dioxolan-4-yl)-2-((4-(4-(trifluoromethoxy)phenoxy)phenyl)sulfonyl)ethyl(hydroxy)formamide, 1,4,5-trideoxy-4-(formyl(hydroxy)amino)-2,3-O-(1-methylethylidene)-5-((4-(4-(trifluoromethoxy)phenoxy)phenyl)sulfonyl)-D-xylitol, (1S)-1-((4S)-2,2-dimethyl-1,3-dioxolan-4-yl)-2-((4-(4-methoxyphenoxy)phenyl)sulfonyl)ethyl(hydroxy)formamide, (1S)-2-((4-(4-chlorophenoxy)phenyl)sulfonyl)-1-((4S)-2,2-dimethyl-1,3-dioxolan-4-yl)ethyl(hydroxy)formamide, (1S)-1-((4S)-2,2-dimethyl-1,3-dioxolan-4-yl)-2-((4-(4-(trifluoromethyl)phenoxy)phenyl)sulfonyl)ethyl(hydroxy)formamide, (1S)-1-((4S)-2,2-dimethyl-1,3-dioxolan-4-yl)-2-((4-(4-methylphenoxy)phenyl)sulfonyl)ethyl(hydroxy)formamide, 4-(((2S)-2-((4S)-2,2-dimethyl-1,3-dioxolan-4-yl)-2-(formyl(hydroxy)amino)ethyl)sulfonyl)-4'-(trifluoromethyl)-1, 1'-biphenyl, (1S)-1-((4S)-1,3-dioxolan-4-yl)-2-((4-(4-(trifluoromethoxy)phenoxy)phenyl)sulfonyl)ethyl(hydroxy)formamide, 4-(((2S)-2-((4S)-2,2-dimethyl-1,3-dioxolan-4-yl)-2-(formyl(hydroxy)amino)ethyl)sulfonyl)-4'-(2-methoxyethoxy)-1,1'-biphenyl, (1S)-1-((4S)-2,2-dimethyl-1,3-dioxolan-4-yl)-2-((4-(4-(2-methoxyethoxy)phenoxy)phenyl)sulfonyl)ethyl(hydroxy)formamide, 1,2,4-trideoxy-2-(formyl(hydroxy)amino)-4,4-dimethyl-3,5-O-(1-methylethylidene)-1-((4-(4-(trifluoromethoxy)phenoxy)phenyl)sulfonyl)-D-threo-pentitol, hydroxy((1S)-1-((2R)-tetrahydro-2-furanyl)-2-((4-(4-(trifluoromethoxy)phenoxy)phenyl)sulfonyl)ethyl)formamide, hydroxy((1R)-1-((2R)-tetrahydro-2-furanyl)-2-((4-(4-(trifluoromethoxy)phenoxy)phenyl)sulfonyl)ethyl)formamide, hydroxy((1S)-1-((2R)-1-(methylsulfonyl)pyrrolidinyl)-2-((4-(4-(trifluoromethoxy)phenoxy)phenyl)sulfonyl)ethyl)formamide, hydroxy((1RS)-1-((4S)-2-oxo-1,3-oxazolidin-4-yl)-2-((4-(4-(trifluoromethoxy)phenoxy)phenyl)sulfonyl)ethyl)formamide, hydroxy((1RS)-1-((4S)-3-methyl-2-oxo-1,3-oxazolidin-4-yl)-2-((4-(4-(trifluoromethoxy)phenoxy)phenyl)sulfonyl)ethyl)formamide, and 1,2-dideoxy-2-(formyl(hydroxy)amino)-3,4-O-(1-methylethylidene)-1-((4-(4-(trifluoromethoxy)phenoxy)phenyl)sulfonyl)-L-threo-pentitol.

20. The compound according to claim 1 which is (1S)-1-((4S)-2,2-dimethyl-1,3-dioxolan-4-yl)-2-((4-(4-(trifluoromethoxy)phenoxy)phenyl)sulfonyl)ethyl(hydroxy)formamide.

## Patentansprüche

1. Eine Verbindung mit der Formel (I), 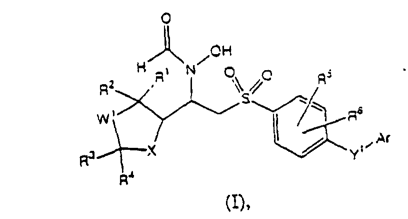 oder ein pharmazeutisch verträgliches Salz oder Prodrug davon,
worin
**W**^{**1**} gewählt ist aus der Gruppe bestehend aus
(1) -O-,
(2) -CH₂O-,
und
(3) -CH₂-;
worin jede Gruppe so gezeichnet ist, dass ihr linkes Ende das Ende ist, das an den Kohlenstoff gebunden ist, der R¹ und R² enthält, und ihr rechtes Ende das Ende ist, das an den Kohlenstoff gebunden ist, der R³ und R⁴ enthält;
**X** ist gewählt aus der Gruppe bestehend aus
(1) -O-,
und
(2)-N(R⁷)-; worin R⁷ gewählt ist aus der Gruppe bestehend aus
(a) Wasserstoff
(b) Alkyl
(c) -SO₂-Alkyl,
und
(d) Alkanoyl;
**R**^{**1**} und **R**^{**2**} sind unabhängig voneinander gewählt aus der Gruppe bestehend aus
(1) Wasserstoff,
(2) Alkyl,
und
(3) Hydroxyalkyl;
**R**^{**3**} und **R**^{**4**} sind unabhängig voneinander gewählt aus der Gruppe bestehend aus
(1) Wasserstoff
und
(2) Alkyl,
oder
**R**^{**3**} und **R**^{**4**} sind zusammengenommen eine Oxogruppe,
oder
**R**^{**3**} und **R**^{**4**} bilden, zusammen mit dem Kohlenstoff, an den sie gebunden sind, einen Cycloalkyl-Ring;
**R**^{**5**} und **R**^{**6**} sind unabhängig voneinander gewählt aus der Gruppe bestehend aus
(1) Wasserstoff,
(2) Alkyl,
(3) Perfluoralkyl,
(4) Halogen,
(5) Halo,
(6) Alkoxy,
(7) Hydroxy,
(8) Hydroxyalkyl,
(9) Alkoxyalkyl,
und
(10) Nitro;
**Y**^{**1**} ist gewählt aus der Gruppe bestehend aus
(1) einer kovalenten Bindung,
(2) -O-
(3) Alkylen von 2 bis 4 Kohlenstoffatomen,
(4) Piperidinyl,
(5) einer Alkenylengruppe aus 2 Kohlenstoffatomen,
(6) einer Alkinylengruppe aus 2 Kohlenstoffatomen,
(7) -SO₂-,
und
(8) -C(O)-;
**Ar** ist gewählt aus der Gruppe bestehend aus
(1) Phenyl,
(2) Pyridyl,
(3) Pyrazinyl,
(4) Pyridazinyl,
(5) Furyl,
(6) Thienyl,
(7) Isoxazolyl,
(8) Oxazolyl,
(9) Thiazolyl,
und
(10) Isothiazolyl,
worin (1)-(10) wahlweise substituiert sein können mit ein, zwei oder drei Substituenten, unabhängig gewählt aus der Gruppe bestehend aus
(a) Alkyl,
(b) Alkoxy, wobei die Alkoxygruppe wahlweise mit Alkoxy substituiert sein kann,
(c) -(Alkylen)-CO₂R₈, wobei R₈ entweder Wasserstoff oder Alkyl ist,
(d) -(Alkylen)-NR₉R₁₀, wobei R₉ und R₁₀ unabhängig voneinander gewählt sind aus der Gruppe bestehend aus
(i) Alkyl,
(ii) Phenyl,
und
(iii) Phenylalkyl,
worin für (ii) und (iii) die Phenylgruppe und der Phenyl-Teil des Phenylalkyls wahlweise mit einem oder zwei Substituenten substiutiert sein können, unabhängig gewählt aus der Gruppe bestehend aus Halo und Alkoxy,
(e) Alkoxyalkyl,
(f) Cyano,
(g) Cyanoalkyl,
(h) Halo,
(i) Haloalkyl,
(j) Hydroxy,
(k) Hydroxyalkyl,
(l) Thioalkoxy,
(m) Thioalkoxyalkyl,
(n) Phenylalkoxy,
(o) Phenoxy,
(p) Phenoxyalkyl,
(q) (Heterozyklus)oxy,
(r) (Heterozyklus)oxyalkyl,
(s) Perfluoralkyl,
(t) Perfluoralkoxy,
(u) Sulfinylalkyl,
(v) Sulfonylalkyl,
(w) ,worin A gewählt ist aus der Gruppe bestehend aus -CH₂-, -CH₂O- und -O-, und B ist gewählt aus der Gruppe bestehend aus -C(O)- und -(C(R'')₂)ᵥ , worin R'' entweder Wasserstoff oder Alkyl ist, und v ist 1-3,
und
(x) -N(R⁸)SO₂R¹¹, worin R¹¹ gewählt ist aus der Gruppe bestehend aus
(i) Wasserstoff,
(ii) Alkyl,
und
(iii) -N(R⁸)(R¹²), worin R¹² Wasserstoff oder Alkyl ist,
worin für (q) und (r) der heterozyklische Teil von (Hetrozyklus)oxy und (Heterozyklus)oxyalkyl gewählt ist aus der Gruppe bestehend aus
(i) Pyridyl,
(ii) Pyrazinyl,
(iii) Pyridazinyl,
(iv) Furyl,
(v) Thienyl,
(vi) Isoxazolyl,
(vii) Oxazolyl,
(viii) Thiazolyl,
und
(ix) Isothiazolyl,
und worin für (q) und (r) der heterozyklische Teil von (Hetrozyklus)oxy und (Heterozyklus)oxyalkyl wahlweise mit einem oder zwei Substituenten substituiert sein kann, unabhängig gewählt aus der Gruppe bestehend aus
(i) Alkyl,
(ii) Alkoxy,
(iii) Perfluoralkyl,
(iv) Halo,
(v) Cyano,
(vi) Cyanoalkyl,
(vii) Haloalkyl,
und
(viii) Alkanoyl, und worin für (o) und (p) der Phenylteil von dem Phenoxy und dem Phenoxyalkyl wahlweise substituiert sein kann mit 1 oder 2 Substituenten, unabhängig gewählt aus der Gruppe bestehend aus
(i) Alkyl,
(ii) Alkoxy,
(iii) Perfluoralkyl,
(iv) Halo,
(v) Cyano,
(vi) Cyanoalkyl,
(vii) Haloalkyl,
und
(viii) Alkanoyl.

2. Eine Verbindung gemäß Anspruch 1, worin Ar Phenyl ist.

3. Eine Verbindung gemäß Anspruch 2, worin Y¹ -O- ist.

4. Eine Verbindung gemäß Anspruch 3, worin W¹ -O- ist.

5. Eine Verbindung gemäß Anspruch 4, worin X -O- ist.

6. Eine Verbindung gemäß Anspruch 5, gewählt aus der Gruppe bestehend aus
(1S)-1-((4S)-2,2-Dimethyl-1,3-dioxolan-4-yl)-2-((4-(4-(trifluormethoxy)phenoxy)phenyl)sulfonyl)ethyl(hydroxy) formamid,
(1R)-1-((4S)-2,2-Dimethyl-1,3-dioxolan-4-yl)-2-((4-(4-(trifluormethoxy)phenoxy)phenyl)sulfonyl)ethyl(hydroxy) formamid,
(1R)-1-((4R)-2,2-Dimethyl-1,3-dioxolan-4-yl)-2-((4-(4-(trifluormethoxy)phenoxy)phenyl)sulfonyl)ethyl(hydroxy) formamid,
(1S)-1-((4R) -2,2-Dimethyl-1,3-dioxolan-4-yl)-2-((4-(4-(trifluormethoxy)phenoxy)phenyl)sulfonyl)ethyl(hydroxy) formamid,
(1S)-1-((4S)-2,2-Dimethyl-1,3-dioxolan-4-yl)-2-((4-(4-(trifluormethoxy)phenoxy)phenyl)sulfonyl)ethyl(hydroxy) formamid,
1,4,5-Trideoxy-4-(formyl(hydroxy)amino)-2,3-O- (1-methylethyliden)-5-((4-(4-(trifluormethoxy)phenoxy)phenyl)sulfonyl)-D-xylitol,
(1S)-1-((4S)-2,2-Dimethyl-1,3-dioxolan-4-yl)-2-((4-(4-(methoxyphenoxy)phenyl)sulfonyl)ethyl(hydroxy)formamid,
(1S)-2-((4-(4-Chlorphenoxy)phenyl)sulfonyl)-1-((4S)-2,2-dimethyl-1,3-dioxolan-4-yl)ethyl(hydroxy)formamid,
(1S)-1-((4S)-2,2-Dimethyl-1,3-dioxolan-4-yl)-2-((4-(4-(trifluormethyl)phenoxy)phenyl)sulfonyl)ethyl(hydroxy)f ormamid,
(1S)-1-((4S)-2,2-Dimethyl-1,3-dioxolan-4-yl)-2-((4-(4-(methylphenoxy)phenyl)sulfonyl)ethyl(hydroxy)formamid,
(1S)-1-((4S)-1,3-Dioxolan-4-yl)-2-((4-(4-(trifluormethoxy)phenoxy)phenyl)sulfonyl)ethyl(hydroxy) formamid,
(1S)-1-((4S)-2,2-Dimethyl-1,3-dioxolan-4-yl)-2-((4-(4-(2-methoxyethoxy)phenoxy)phenyl)sulfonyl)ethyl(hydroxy)formamid, und
1,2-Dideoxy-2-(formyl(hydroxy)amino)-3,4-O- (1-methylethyliden)-1-((4-(4-(trifluormethoxy)phenoxy)phenyl)sulfonyl)-L-threopentitol.

7. Eine Verbindung gemäß Anspruch 4, worin X -N(R⁷)- ist.

8. Eine Verbindung gemäß Anspruch 7, gewählt aus der Gruppe bestehend aus Hydroxy((1RS)-1-((4S)-2-oxo-1,3-oxazolidin-4-yl)-2-((4-(4-(trifluormethoxy)phenoxy)phenyl)sulfonyl)ethyl)formamid und
Hydroxy((1RS)-1-((4S)-3-methyl-2-oxo-1,3-oxazolidin-4-yl)-2-((4-(4-(trifluormethoxy)phenoxy)phenyl)sulfonyl)ethyl)formami.

9. Eine Verbindung gemäß Anspruch 3, worin W¹ -CH₂- ist.

10. Eine Verbindung gemäß Anspruch 9, worin X -O- ist.

11. Eine Verbindung gemäß Anspruch 10, gewählt aus der Gruppe bestehend aus Hydroxy((1S)-1-((2R)-tetrahydro-2-furanyl)-2-((4-(4-(trifluormethoxy)phenoxy)phenyl)sulfonyl)ethyl)formamid und
Hydroxy((1R)-1-((2R)-tetrahydro-2-furanyl)-2-((4-(4-(trifluormethoxy)phenoxy)phenyl)sulfonyl)ethyl)formamid

12. Eine Verbindung gemäß Anspruch 9, worin X -N(R⁷)- ist.

13. Eine Verbindung gemäß Anspruch 12, die Hydroxy((1S)-1-((2R)-1-(methylsulfonyl)pyrrolidinyl)-2-((4-(4-(trifluormethoxy)phenoxy)phenyl)sulfonyl)ethyl)formamid ist.

14. Eine Verbindung gemäß Anspruch 3, in der W -CH₂O- ist.

15. Eine Verbindung gemäß Anspruch 14, die 1,2,4-Trideoxy-2-(formyl(hydroxy)amino)-4,4-dimethyl-3,5-O-(1-methylethyliden).- . 1-((4-(4-(trifluormethoxy)phenoxy)phenyl)sulfonyl)-D-threopentitol ist.

16. Eine Verbindung gemäß Anspruch 2, in der Y eine kovalente Bindung ist, W¹ -O- ist und X -O- ist.

17. Eine Verbindung gemäß Anspruch 16, bestehend aus 4-Chlor-4'-(((2S)-2-((4S)-2,2-dimethyl-1,3-dioxolan-4-yl)-2-(formyl(hydroxy)amino)ethyl)sulfonyl)-1,1'-biphenyl, 4-(((2S)-2-((4S)-2,2-Dimethyl-1,3-dioxolan-4-yl)-2-(formyl(hydroxy)amino)ethyl)sulfonyl)-4'-(trifluormethyl)-1,1'-biphenyl
und
4-(((2S)-2-((4S)-2,2-Dimethyl-1,3-dioxolan-4-yl)-2-(formyl(hydroxy)amino)ethyl)sulfonyl)-4'-(2-methoxyethoxy)-1,1'-biphenyl.

18. Verwendung einer Verbindung des Anspruchs 1 zur Herstellung eines Medikaments zur Hemmung der Matrix-Metallproteinase in einem Säugetier, bei erkanntem Bedarf an einer derartigen Behandlung.

19. Eine Verbindung gemäß Anspruch 1, gewählt aus der Gruppe bestehend aus
(1S)-1-((4S)-2,2-Dimethyl-1,3-dioxolan-4-yl)-2-((4-(4-(trifluormethoxy)phenoxy)phenyl)sulfonyl)ethyl(hydroxy) formamid,
(1R)-1-((4S)-2,2-Dimethyl-1,3-dioxolan-4-yl)-2-((4-(4-(trifluormethoxy)phenoxy)phenyl)sulfonyl)ethyl(hydroxy) formamid,
(1R)-1-((4R)-2,2-Dimethyl-1,3-dioxolan-4-yl)-2-((4-(4-(trifluormethoxy)phenoxy)phenyl)sulfonyl)ethyl(hydroxy) formamid,
(1S)-1-((4R)-2,2-Dimethyl-1,3-dioxolan-4-yl)-2-((4-(4-(trifluormethoxy)phenoxy)phenyl)sulfonyl)ethyl(hydroxy) formamid,
4-Chlor-4'-(((2S)-2-((4S)-2,2-dimethyl-1,3-dioxolan-4-yl)-2-(formyl(hydroxy)amino)ethyl)sulfonyl)-1,1'-biphenyl,
(1S)-1-((4S)-2,2-Diethyl-1,3-dioxolan-4-yl)-2-((4-(4-(trifluormethoxy)phenoxy)phenyl)sulfonyl)ethyl(hydroxy) formamid,
1,4,5-Trideoxy-4-(formyl(hydroxy)amino)-2,3-O-(1-methylethyliden)-5-((4-(4-(trifluormethoxy)phenoxy)phenyl)sulfonyl)-D-xylitol,
(1S)-1-((4S)-2,2-Dimethyl-1,3-dioxolan-4-yl)-2-((4-(4-methoxyphenoxy)phenyl)sulfonyl)ethyl(hydroxy)formamid,
(1S)-2-((4-(4-Chlorphenoxy)phenyl)sulfonyl)-1-((4S)-2,2-dimethyl-1,3-dioxolan-4-yl)ethyl(hydroxy)formamid,
(1S)-1-((4S)-2,2-Dimethyl-1,3-dioxolan-4-yl)-2-((4-(4-(trifluormethyl)phenoxy)phenyl)sulfonyl)ethyl(hydroxy)f ormamid,
(1S)-1-((4S)-2,2-Dimethyl-1,3-dioxolan-4-yl)-2-((4-(4-methylphenoxy)phenyl)sulfonyl)ethyl(hydroxy)formamid,
4-(((2S)-2-((4S)-2,2-Dimethyl-1,3-dioxolan-4-yl)-2-(formyl(hydroxy)amino)ethyl)sulfonyl-4'-(trifluormethyl)-1,1'biphenyl,
(1S)-1-((4S)-1,3-Dioxolan-4-yl)-2-((4-(4-(trifluormethoxy)phenoxy)phenyl)sulfonyl)ethyl(hydroxy) formamid,
4-(((2S)-2-((4S)-2,2-Dimethyl-1,3-dioxolan-4-yl)-2-(formyl(hydroxy)amino)ethyl)sulfonyl)-4'-(2-methoxyethoxy)-1,1'-biphenyl,
(1S)-1-((4S)-2,2-Dimethyl-1,3-dioxolan-4-yl)-2-((4-(4-(2-methoxyethoxy)phenoxy)phenyl)sulfonyl)ethyl(hydroxy)formamid,
1,2,4-Trideoxy-2-(formyl(hydroxy)amino)-4,4-dimethyl-3,5-O-(1-methylethyliden)-1-((4-(4-(trifluormethoxy)phenoxy)phenyl)sulfonyl)-D-threopentitol,
Hydroxy((1S)-1-((2R)-tetrahydro-2-furanyl)-2-((4-(4-(trifluormethoxy)phenoxy)phenyl)sulfonyl)ethyl)formamid
Hydroxy((1R)-1-((2R)-tetrahydro-2-furanyl)-2-((4-(4-(tifluormethoxy)phenoxy)phenyl)sulfonyl)ethyl)formamid,
Hydroxy((1S)-1-((2R)-1-(methylsulfonyl)pyrrolidinyl)-2-((4-(4-(trifluormethoxy)phenoxy)phenyl)sulfonyl)ethyl)formamid, Hydroxy((1RS)-1-((4S)-2-oxo-1,3-oxazolidin-4-yl)-2-((4-(4-(trifluormethoxy)phenoxy)phenyl)sulfonyl)ethyl)formamid ,
Hydroxy((1RS)-1-((4S)-3-methyl-2-oxo-1,3-oxazolidin-4-yl)-2-((4-(4-(trifluormethoxy)phenoxy)phenyl)sulfonyl)ethyl)formamid,
und
1,2-Dideoxy-2-(formyl(hydroxy)amino)-3,4-O-(1-methylethyliden)-1-((4-(4-(trifluormethoxy)phenoxy)phenyl)sulfonyl)-L-threopentitol.

20. Eine Verbindung gemäß Anspruch 1, die
(1S)-1-((4S)-2,2-Dimethyl-1,3-dioxolan-4-yl)-2-((4-(4-(trifluormethoxy)phenoxy)phenyl)sulfonyl)ethyl(hydroxy) formamid ist.

## Revendications

1. Composé de formule (I) 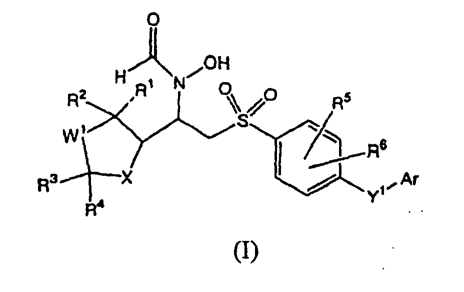 ou un de ses sels ou un de ses promédicaments, où
**W**^{**1**} est choisi dans le groupe constitué par
(1) -O-,
(2) -CH₂O-, et
(3) -CH₂-;
chaque groupe étant représenté de façon que l'extrémité gauche soit l'extrémité qui se lie au carbone contenant R¹ et R² et que l'extrémité droite soit l'extrémité qui se lie au carbone contenant R³ et R⁴;
**X** est choisi dans le groupe constitué par
(1)-O-, et
(2) -N(R⁷)-, où R⁷ est choisi dans le groupe constitué par
(a) l'hydrogène,
(b) un radical alkyle,
(c) un radical -SO₂-alkyle, et
(d) un radical alcanoyle;
**R**^{**1**} et **R**^{**2**} sont choisis indépendamment dans le groupe constitué par
(1) l'hydrogène,
(2) un radical alkyle, et
(3) un radical hydroxyalkyle;
**R**^{**3**} et **R**^{**4**} sont choisis indépendamment dans le groupe constitué par
(1) l'hydrogène, et
(2) un radical alkyle,
ou
**R**^{**3**} et **R**^{**4**} pris ensemble forment un radical oxo, ou
**R**^{**3**} et **R**^{**4**}, pris ensemble avec l'atome de carbone auquel ils sont liés, forment un cycle cycloalkyle;
**R**^{**5**} et **R**^{**6**} sont choisis indépendamment dans le groupe constitué par
(1) l'hydrogène,
(2) un radical alkyle,
(3) un radical perfluoroalkyle,
(4) un radical halogéno,
(5) un radical halogénoalkyle,
(6) un radical alcoxy,
(7) un radical hydroxy,
(8) un radical hydroxyalkyle,
(9) un radical alcoxyalkyle, et
(10) un radical nitro;
**Y**^{**1**} est choisi dans le groupe constitué par
(1) une liaison covalente,
(2) -O-,
(3) un radical alkylène de 2 à 4 atomes de carbone,
(4) un radical pipéridinényle,
(5) un radical alcénylène de 2 atomes de carbone,
(6) un radical alcynylène de 2 atomes de carbone,
(7) -SO₂- et
(8) -CO-;
**Ar** est choisi dans le groupe constitué par les radicaux
(1) phényle,
(2) pyridyle,
(3) pyrazinyle,
(4) pyridazinyle,
(5) furyle,
(6) thiényle,
(7) isoxazolyle,
(8) oxazolyle,
(9) thiazolyle et
(10) isothiazolyle,
les groupes (1) à (10) pouvant être éventuellement substitués par 1, 2 ou 3 substituants choisis indépendamment dans le groupe constitué par les radicaux
(a) alkyle,
(b) alcoxy, le radical alcoxy pouvant éventuellement être substitué par alcoxy,
(c) -(alkylène)-CO₂R₈, où R₈ est un atome d'hydrogène ou un radical alkyle,
(d) -(alkylène)-NR₉R₁₀, où R₉ et R₁₀ sont choisis indépendamment dans le groupe constitué par les radicaux
(i) alkyle,
(ii) phényle et
(iii) phénylalkyle,
où, en ce qui concerne (ii) et (iii), le radical phényle et la partie phényle du radical phénylalkyle peuvent éventuellement être substitués par 1 ou 2 substituants choisis dans le groupe constitué par les radicaux halogéno et alcoxy,
(e) alcoxyalkyle,
(f) cyano,
(g) cyanoalkyle,
(h) halogéno,
(i) halogénoalkyle,
(j) hydroxy,
(k) hydroxyalkyle,
(l) thioalcoxy,
(m) thioalcoxyalkyle,
(n) phénylalcoxy,
(o) phénoxy,
(p) phénoxyalkyle,
(q) (hétérocycle)alkyle,
(r) (hétérocycle)oxyalkyle,
(s) perfluoroalkyle,
(t) perfluoroalcoxy,
(u) sulfonylalkyle,
(w) 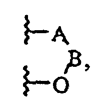 où A est choisi dans le groupe constitué par -CH₂-, -CH₂O- et -O-, et B est choisi dans le groupe constitué par -C(O)- et -(C(R")₂)ᵥ-,
où R" est un atome d'hydrogène ou un radical alkyle, et v est égal à 1-3, et
(x) -N(R⁸)SO₂R¹¹ où R¹¹ est choisi dans le groupe constitué par
(i) l'hydrogène,
(ii) un radical alkyle, et
(iii) un radical -N(R⁸)(R¹²) dans lequel R¹² est un atome d'hydrogène ou un radical alkyle,
où, en ce qui concerne (q) et (r), la partie hétérocycle des groupes (hétérocycle)oxy et (hétérocycle)oxyalkyle est choisie dans le groupe constitué par les radicaux
(i) pyridyle,
(ii) pyrazinyle,
(iii) pyridazinyle,
(iv) furyle,
(v) thiényle,
(vi) isoxazolyle,
(vii) oxazolyle,
(viii) thiazolyle et
(ix) isothiazolyle,
et où, en ce qui concerne (q) et (r), la partie hétérocycle des radicaux (hétérocycle)oxy et (hétérocycle)oxyalkyle peut éventuellement être substituée par 1 ou 2 substituants choisis indépendamment dans le groupe constitué par les radicaux
(i) alkyle,
(ii) alcoxy,
(iii) perfluoroalkyle,
(iv) halogéno,
(v) cyano,
(vi) cyanoalkyle,
(vii) halogénoalkyle, et
(viii) alcanoyle,
et où, en ce qui concerne (o) et (p), la partie phényle des radicaux phénoxy et phénoxyalkyle peut être éventuellement substituée par 1 ou 2 substituants choisis indépendamment dans le groupe constitué par les radicaux
(i) alkyle,
(ii) alcoxy,
(iii) perfluoroalkyle,
(iv) halogéno,
(v) cyano,
(vi) cyanoalkyle,
(vii) halogénoalkyle, et
(viii) alcanoyle.

2. Composé selon la revendication 1, dans lequel Ar est un radical phényle.

3. Composé selon la revendication 2, dans lequel Y¹ est -O-.

4. Composé selon la revendication 3, dans lequel W¹ est -O-.

5. Composé selon la revendication 4, dans lequel X est -O-.

6. Composé selon la revendication 5, choisis dans le groupe constitué par le 1-((4S)-2,2-diméthyl-1,3-dioxolan-4-yl)-2-((4-(4-(trifluorométhoxy)phénoxy)-phényl)sulfonyl)éthyl(hydroxy)formamide, le (1R)-1-((4S)-2,2-diméthyl-1,3-dioxolan-4-yl)-2-((4-(4-(trifluorométhoxy)-phénoxy)phényl)sulfonyl)éthyl(hydroxy)formamide, le (1R)-1-((4R)-2,2-diméthyl-1,3-dioxolan-4-yl)-2-((4-(4-(trifluorométhoxy)-phénoxy)phényl)sulfonyl)éthyl(hydroxy)formamide, le (1S)-1-((4R)-2,2-diméthyl-1,3-dioxolan-4-yl)-2-((4-(4-(trifluorométhoxy)-phénoxy)phényl)sulfonyl)éthyl(hydroxy)formamide, le (1S)-1-((4S)-2,2-diéthyl-1,3-dioxolan-4-yl)-2-((4-(4-(trifluorométhoxy)phénoxy)-phényl)sulfonyl)éthyl(hydroxy)formamide, le 1,4,5-tridésoxy-4-(formyl(hydroxy)amino)-2,3-O-(1-méthyléthylidène)-5-((4-(4-(trifluorométhoxy)phénoxy)phényl)sulfonyl)-D-xylitol, le 1-((4S)-2,2-diméthyl-1,3-dioxolan-4-yl)-2-((4-(4-(trifluorométhoxy)phénoxy)-phényl)sulfonyl)éthyl(hydroxy)formamide, le (1R)-1-((4S)-2,2-diméthyl-1,3-dioxolan-4-yl)-2-((4-(4-(trifluorométhoxy)-phénoxy)phényl)sulfonyl)éthyl(hydroxy)formamide, le (1R)-1-((4R)-2,2-diméthyl-1,3-dioxolan-4-yl)-2-((4-(4-(trifluorométhoxy)-phénoxy)phényl)sulfonyl)éthyl(hydroxy)formamide, le (1S)-1-((4R)-2,2-diméthyl-1,3-dioxolan-4-yl)-2-((4-(4-(trifluorométhoxy)-phénoxy)phényl)sulfonyl)éthyl(hydroxy)formamide, le (1S)-1-((4S)-2,2-diéthyl-1,3-dioxolan-4-yl)-2-((4-(4-(trifluorométhoxy)-phénoxy)phényl)sulfonyl)éthyl(hydroxy)formamide, le 1,4,5-tridésoxy-4-(formyl(hydroxy)amino)-2,3-O-(1-méthyléthylidène)-5-((4-(4-(trifluorométhoxy)phénoxy)phényl)sulfonyl)-D-xylitol, le (1S)-1-((4S)-2,2-diméthyl-1,3-dioxolan-4-yl)-2-((4-(4-méthoxyphénoxy)phényl)-sulfonyl)éthyl(hydroxy)formamide, le (1S)-2-((4-(4-chlorophénoxy)phényl)sulfonyl)-1-((4S)-2,2-diméthyl-1,3-dioxolan-4-yl)éthyl(hydroxy)formamide, le (1S)-1-((4S)-2,2-diméthyl-1,3-dioxolan-4-yl)-2-((4-(4-(trifluorométhyl)phénoxy)-phényl)sulfonyl)éthyl(hydroxy)formamide, le (1S)-1-((4S)-2,2-diméthyl-1,3-dioxolan-4-yl)-2-((4-(4-méthylphénoxy)phényl)-sulfonyl)éthyl(hydroxy)formamide, le (1S)-1-((4S)-1,3-dioxolan-4-yl)-2-((4-(4-(trifluorométhoxy)phénoxy)phényl)-sulfonyl)éthyl(hydroxy)formamide, le (1S)-1((4S)-2,2-diméthyl-1,3-dioxolan-4-yl)-2-((4-(4-(2-méthoxyéthoxy)-phénoxy)phényl)sulfonyl)éthyl(hydroxy)formamide, et le 1,2-didésoxy-2-(formyl(hydroxy)amino)-3,4-O-(1-méthyléthylidène)-1-((4-(4-(trifluorométhoxy)phénoxy)phényl)sulfonyl)-L-thréo-pentitol.

7. Composé selon la revendication 4, dans lequel X est -N(R⁷)-.

8. Composé selon la revendication 7, choisi dans le groupe constitué par l'hydroxy((1RS)-1-((4S)-2-oxo-1,3-oxazolidin-4-yl)-2-((4-(4-(trifluorométhoxy)-phénoxy)phényl)sulfonyl)éthyl)formamide et l'hydroxy((1RS)-1-((4S)-3-méthyl-2-oxo-1,3-oxazolidin-4-yl)-2-((4-(4-(trifluorométhoxy)phénoxy)phényl)sulfonyl)éthyl)formamide.

9. Composé selon la revendication 3, dans lequel W¹ est -CH₂-.

10. Composé selon la revendication 9, dans lequel X est -O-.

11. Composé selon la revendication 10, choisi dans le groupe constitué par l'hydroxy((1S)-1-((2R)-tétrahydro-2-furanyl)-2-((4-(4-(trifluorométhoxy)phénoxy)-phényl)sulfonyl)éthyl)formamide et l'hydroxy((1R)-1-((2R)-tétrahydro-2-furanyl)-2-((4-(4-(trifluorométhoxy)phénoxy)-phényl)sulfonyl)éthyl)formamide.

12. Composé selon la revendication 9, dans lequel X est -N(R⁷)-.

13. Composé selon la revendication 12, qui est l'hydroxy((1S)-1-((2R)-1-(méthylsulfonyl)pyrrolidinyl)-2-((4-(4-(trifluorométhoxy)-phénoxy)phényl)sulfonyl)éthyl)formamide.

14. Composé selon la revendication 3, dans lequel W est -CH₂O-.

15. Composé selon la revendication 14, qui est le 1,2,4-tridésoxy-2-(formyl(hydroxy)amino)-4,4-diméthyl-3,5-O-(1-méthyléthylidène)-1-((4-(4-(trifluorométhoxy)phénoxy)phényl)sulfonyl)-D-thréo-pentitol.

16. Composé selon la revendication 2, dans lequel Y est une liaison covalente, W¹ est -O- et X est -O-.

17. Composé selon la revendication 16, constitué par le 4-chloro-4'-(((2S)-2-((4S)-2,2-diméthyl-1,3-dioxolan-4-yl)-2-(formyl(hydroxy)-amino)éthyl)sulfonyl)-1,1'-biphényle, le 4-(((2S)-2-((4S)-2,2-diméthyl-1,3-dioxolan-4-yl)-2-(formyl(hydroxy)amino)éthyl)-sulfonyl)-4'-trifluorométhyl)-1,1'-biphényle, et le 4-(((2S)-2-((4S)-2,2-diméthyl-1,3-dioxolan-4-yl)-2-(formyl(hydroxy)amino)-éthyl)sulfonyl)-4'-(2-méthoxyéthoxy)-1,1'-biphényle.

18. Utilisation d'un composé de la revendication 1 pour la préparation d'un médicament destiné à inhiber la métalloprotéase matricielle chez un mammifère ayant un besoin reconnu d'un tel traitement.

19. Composé selon la revendication 1, choisi dans le groupe constitué par le (1S)-1 -((4S)-2,2-diméthyl-1,3-dioxolan-4-yl)-2-((4-(4-(trifluorométhoxy)-phénoxy)phényl)sulfonyl)éthyl(hydroxy)formamide, le (1R)-1 -((4S)-2,2-diméthyl-1,3-dioxolan-4-yl)-2-((4-(4-(trifluorométhoxy)-phénoxy)phényl)sulfonyl)éthyl(hydroxy)formamide, le (1R)-1-((4R)-2,2-diméthyl-1,3-dioxolan-4-yl)-2-((4-(4-(trifluorométhoxy)-phénoxy)phényl)sulfonyl)éthyl(hydroxy)formamide, le (1S)-1-((4R)-2,2-diméthyl-1,3-dioxolan-4-yl)-2-((4-(4-(trifluorométhoxy)-phénoxy)phényl)sulfonyl)éthyl(hydroxy)formamide, le 4-chloro-4'-(((2S)-2-((4S)-2,2-diméthyl-1,3-dioxolan-4-yl)-2-(formyl(hydroxy)-amino)éthyl)sulfonyl)-1,1'-biphényle, le (1S)-1-((4S)-2,2-diéthyl-1,3-dioxolan-4-yl)-2-((4-(4-(trifluorométhoxy)-phénoxy)phényl)sulfonyl)éthyl(hydroxy)formamide, le 1,4,5-tridésoxy-4-(formyl(hydroxy)amino)-2,3-O-(1-méthyléthylidéne)-5-((4-(4-(trifluorométhoxy)phénoxy)phényl)sulfonyl)-D-xylitol, le (1S)-1-((4S)-2,2-diméthyl-1,3-dioxolan-4-yl)-2-((4-(4-méthoxyphénoxy)phényl)-sulfonyl)éthyl(hydroxy)formamide, le (1S)-2-((4-(4-chlorophénoxy)phényl)sulfonyl)-1-((4S)-2,2-diméthyl-1,3-dioxolan-4-yl)éthyl(hydroxy)formamide, le (1S)-1-((4S)-2,2-diméthyl-1,3-dioxolan-4-yl)-2-((4-(4-(trifluorométhyl)-phénoxy)phényl)sulfonyl)éthyl(hydroxy)formamide, le (1S)-1-((4S)-2,2-diméthyl-1,3-dioxolan-4-yl)-2-((4-(4-méthylphénoxy)phényl)-sulfonyl)éthyl(hydroxy)formamide, le 4-(((2S)-2-((4S)-2,2-diméthyl-1,3-dioxolan-4-yl)-2-(formyl(hydroxy)amine)-éthyl)sulfonyl)-4'-(trifluorométhyl)-1,1'-biphényle, le (1S)-1-((4S)-1,3-dioxolan-4-yl)-2-((4-(4-(trifluorométhoxy)phénoxy)phényl)-sulfonyl)éthyl(hydroxy)formamide, le 4-(((2S)-2-((4S)-2,2-diméthyl-1,3-dioxolan-4-yl)-2-(formyl(hydroxy)amino)-éthyl)sulfonyl)-4'-(2-méthoxyéthoxy)-1,1'-biphényle, le (1S)-1-((4S)-2,2-diméthyl-1,3-dioxolan-4-yl)-2-((4-(4-(2-méthoxyéthoxy)-phénoxy)phényl)sulfonyl)éthyl(hydroxy)formamide, le 1,2,4-tridésoxy-2-(formyl(hydroxy)amino)-4,4-diméthyl-3,5-O-(1-méthyléthylidène)-1-((4-(4-(trifluorométhoxy)phénoxy)phényl)sulfonyl)-D-thréo-pentitol, l'hydroxy(( 1S)-1-((2R)-tétrahydro-2-furanyl)-2-((4-(4-(trifluorométhoxy)phénoxy)-phényl)sulfonyl)éthyl)formamide, l'hydroxy((1 R)-1 -((2R)-tétrahydro-2-furanyl)-2-((4-(4-(trifluorométhoxy)phénoxy)-phényl)sulfonyl)éthyl)formamide, l'hydroxy((1S)-1-((2R)-1-(méthylsulfonyl)pyrrolidinyl)-2-((4-(4-(trifluorométhoxy)phénoxy)phényl)sulfonyl)éthyl)formamide, l'hydroxy((1RS)-1-((4S)-2-oxo-1,3-oxazolidin-4-yl)-2-((4-(4-(trifluorométhoxy)-phénoxy)phényl)sulfonyl)éthyl)formamide, l'hydroxy((1RS)-1-((4S)-3-méthyl-2-oxo-1,3-oxazolidin-4-yl)-2-((4-(4-(trifluorométhoxy)phénoxy)phényl)sulfonyl)éthyl)formamide, et le 1,2-didésoxy-2-(formyl(hydroxy)amino)-3,4-O-(1-méthyléthylidène)-1-((4-(4-(trifluorométhoxy)phénoxy)phényl)sulfonyl)-L-thréo-pentitol.

20. Composé selon la revendication 1, qui est le (1S)-1-((4S)-2,2-diméthyl-1,3-dioxolan-4-yl)-2-((4-(4-(trifluorométhoxy)-phénoxy)phényl)sulfonyl)éthyl(hydroxy)formamide.
